# EUROPEAN PATENT SPECIFICATION

(11) **EP 1 853 291 B1**
(45) Date of publication and mention of the grant of the patent: **05.05.2010**
(21) Application number: 05772001.3
(22) Date of filing: 20.07.2005
(51) Int. Cl.: A61K 38/02

(54) **BACTERIOPHAGE AND PROPHAGE PROTEINS IN CANCER GENE THERAPY**
BAKTERIOPHAGEN- UND PROPHAGEN-PROTEINE IN DER KREBSGENTHERAPIE
PROTÉINES DE BACTÉRIOPHAGES AND PROPHAGES DANS LA THÉRAPIE GÉNIQUE DU CANCER

(30) Priority: 20.07.2004 EP 04017128
(43) Date of publication of application: 14.11.2007
(73) Proprietor: Ziel Biopharma Ltd, Corbally Limerick (IE)
(72) Inventor: BLÄSI, Udo, A-3464 Hausleiten (AT); HOHENADL, Christine, A-1200 Wien (AT)
(74) Representative: Fleuchaus, Andrea
(86) International application number: PCT/EP2005/053533
(87) International publication number: WO 2006/008312

(56) References cited:
- WO-A-01/90331
- WO-A-03/025154
- WO-A-2004/013317
- WO-A-2004/046319

## Description

### FIELD OF THE INVENTION

The present invention relates to the use of a polypeptide having a proliferation inhibitory activity or a cell death inducing activity on animal cells. The invention provides further means and methods to use said polypeptide and the corresponding nucleic acids sequence containing the coding regions of said polypeptide. The invention further relates to vectors expressing said polypeptide, to pharmaceutical compositions and the polypeptide for use in the treatment of proliferative disorders or diseases like cancer. Last but not least the invention provides an in vitro method for a gene therapeutic approach using said polypeptide having a proliferation inhibitory activity or a cell death inducing activity on animal cells.

### BACKGROUND OF THE INVENTION

Development of cancer as a cause of death is a growing problem in populations with good health care and a high percentage of aged people. So far, limited options, like surgery or chemo- and radiotherapy, exist to treat cancer or to cure patients. This is one reason why gene therapy approaches have evolved primarily concentrating on treating cancer.

One gene therapy approach makes use of so-called suicide genes. A suicide gene is a gene whose expression in a cell is lethal for that cell.

Most suicide genes mediate a cell killing effect by coding for viral, bacterial, fungal, plant or human enzymes that convert a chemical substance with low inherent toxicity into a cytotoxic compound [1]. This approach is also referred to as gene-directed enzyme prodrug therapy (GDEPT) or virus-directed enzyme prodrug therapy (VDEPT) if viral vectors are used to deliver the gene.

Most enzymes encoded by such prodrug converting or conditionally toxic suicide genes mediate toxicity through disruption of DNA replication while the expressed product of the suicide gene induces the conversion of a prodrug into its toxic metabolites, which then act on replicating DNA [1, 6]. Some of the best-studied enzymes are the Herpes simplex virus (HSV)-1 thymidine kinase [2], cytosine deaminase [3] from bacteria or yeast, bacterial nitroreductase [56] and the mammalian liver enzyme cytochrome P450 [4, 5]. Once these enzymes are expressed in the target cell, the respective prodrug (ganciclovir, fluorocytosine, CB1954, and cyclophosphamide or ifosfamide, respectively) can be applied systemically and will be activated only in cells, which carry and express the respective suicide gene.

In another approach the plant enzyme gene linamarase *(lis*) hydrolyses the cyanogenic glucoside substrate, linamarin (lin), into glucose and the cell toxic cyanide [57].

The drawbacks of these approaches are the systemic delivery of the prodrug implicating high doses which may provoke side effects, especially in the case of cytochrome P450, which is physiologically expressed predominantly in cells of the liver.

Another drawback can be seen in the development of cancer cell resistance, which develops either to the prodrug or to cell killing, which in this kind of therapy is usually due to apoptosis [7, 8] and thus to depletion of cells carrying the respective suicide gene.

Another strategy makes use of genes encoding for cytotoxic proteins which have a direct lethal effect on cells such as fusogenic membrane proteins like *Vesicular Stomatitis Virus* (VSV) glycoprotein or *Gibbon Ape Leukemia Virus* (GALV) envelope protein [9-11]. Fusogenic membrane proteins, once expressed in a target cell, cause membrane fusions of neighbouring cells leading to the formation of large multinuclear syncytia, which finally die either by apoptosis or necrosis [12-14]. Other toxins that have been used for direct cell killing are e.g. *Corynebacterium diphtheria* toxin A [15-18], Cholera toxin B [19, 20], Anthrax (*Bacillus anthracis*) toxin [21-24], Pseudomonas (*Pseudomonas aeruginosa*) toxin [58], or Ricin (*Ricinus communis*) [58].

Still another approach makes use of so called apoptosis inducers to activate apoptotic pathways and to induce cell death. The genes used in these studies are e.g. caspase-1/interieukin-1β-converting enzyme (ICE) [59], caspase-3/CPP32β [60], caspase-6 [61], caspase-8 [62], Fas associated protein with death domain (FADD) [63] and Bax [64].

Suicide genes must be introduced into cells in ways that ensure their uptake and expression by as many target cells as possible, while limiting their expression by non-target cells. Suicide gene therapy for cancer ideally requires a vector having the capacity to discriminate between target and non-target cells.

Considering viral vectors (for review see [25]), a tightly controlled production system is required when toxic genes are encoded to protect the respective packaging cells [26] from premature killing.

Although some cytotoxic suicide genes are already known, there is still a need for further and alternative suicide genes having advantageous characteristics, particularly having the capacity to kill or eradicate target cells, especially cancer cells in a patient.

It is thus an object of the present invention to provide a new set of cytotoxic suicide genes and their corresponding cytotoxic proteins or polypeptides having a cell growth inhibitory and/or cell death inducing activity in eukaryotic, particularly animal or mammalian cells including human cells.

### DETAILED DESCRIPTION OF THE INVENTION

Here we show that bacteriophage- and pro-phage-derived alpha-helix-type channel forming proteins, in particular holin proteins, which naturally cause lysis of prokaryotic cells through pore formation in the cytoplasmic membrane, causes a significant cell growth inhibitory or even a cell death inducing activity when introduced and/or expressed in eukaryotic cells, particularly animal or human cells.

In the context of the invention the term "animal" or "animal cells" is used for organism or cells of organism belonging to the kingdom of Animalia according to the current five kingdom system of classification (Margulis & Schwartz, 1998. *Five Kingdoms. An Illustrated Guide to the Phyla of Life on Earth*., W.H. Freeman, New York) which comprises the kingdoms Animalia, Plantae, Protista, Fungi and Monera (comprising the prokaryotic bacteria and cyanobacteria). Accordingly, the term "animal cells" also comprises "human" cells. However, for reasons of intensification in several occasions during the application human cells are additionally mentioned. In the context of the invention "animal or human cells" could be located within a tissue or within an animal or human body (*"in-vivo*") or could be isolated from its natural environment e.g. cell lines, primary cells ("*in-vitro*").

The term "proliferative disease" or "proliferative disorder" characterized by an abnormal proliferation of cells is used herein in a broad sense to include any disorder that requires control of the cell cycle, for example cancers and leukaemias but also cardiovascular disorders such as restenosis and cardiomyopathy, auto-immune disorders such as glomerulonephritis and rheumatoid arthritis, dermatological disorders such as psoriasis, anti-inflammatory, anti-fungal, antiparasitic disorders such as malaria, emphysema and alopecia. In these disorders, the bacteriophage-derived protein, preferably a holin of the present invention could be used to induce cell death or maintain stasis within the target cell population.

Bacteriophage-derived "holins" belong to a functional superfamily consisting of at least sixteen distinct families of proteins that exhibit common structural and functional characteristics. More particular, bacteriophage-encoded holins are members of the α-helix type of channel-forming proteins and are required for the lysis of the infected bacterial cells and as a consequence, for the release of bacteriophage particles. However, the members of that family do not exhibit a statistically significant sequence similarity or homology.

In the context of the present invention the term "bacteriophage" comprises bacteriophages per se as well as pro-phages, which are bacteriophages that are in a dormant state or which are even defective. Its genome is either integrated into that of the host bacterium, or is replicated autonomously.

Holins are encoded by genes of pro-phages of Gram-positive and Gram-negative bacteria as well as bacteriophages associated with these organisms [28]. The primary function of holins appear to be the transport of murein hydrolases across the cytoplasmic membrane to the cell wall where these enzymes hydrolyze bonds in the peptidoglycan cell wall polymer as a prelude to cell lyses. When chromosomally encoded, these enzymes are therefore autolysins. Holins may also facilitate leakage of electrolytes and nutrients from the cell cytoplasm, thereby promoting cell death. Murein hydrolases lack N-terminal signal sequences, and therefore are not believed to be transported via the general secretory pathway. Holins undoubtedly form oligomeric complexes that generate pores in the cytoplasmic membrane. These pores are thought to provide a passive transport pathway for their substrate proteins.

It was highly surprising for the inventors to find out, that such holins, when they are expressed in human or animal cells induce cell killing or inhibit cell growth in these cells. These results were particularly surprising, since eukaryotic cell metabolism is completely different to the metabolism of bacterial systems. Additionally also the structure and organization of a eukaryotic cell is fundamentally different from a bacterial cell and moreover eukaryotic cells are not hosts for bacteriophages. Accordingly, it was not predictable, whether holins could be expressed in an animal cell or human cell, what pathways they would use, accordingly where in the cellular compartments such proteins would be localized, whether their functionality would be preserved or modified, whether the cellular metabolism would be affected or activated, and last but not least what effects the introduction and expression of such holins in an eukaryotic cell would have.

While answering these and further basic research questions the inventors were able to provide means and methods to use the bacteriophage-derived holins for treating proliferative diseases and cancer. In particular they provide means and methods for a gene therapeutical approach of treating cancer.

In a preferred embodiment of the invention the bacteriophage-derived proteins are alpha-helix-type channel forming proteins. These proteins are selected, but not limited to double stranded DNA bacteriophages, single stranded DNA bacteriophages or single stranded RNA bacteriophages, more preferably they are selected from the group consisting of the Lambda S105 and Lambda S107 protein (Swiss-Prot Accession Number P03705), the Phi29 GP 14 protein (Swiss-Prot Accession Number P11188), the phage T4 Immunity protein (Swiss-Prot Accession Number P08986), the phage P22 protein 13 (Swiss-Prot Accession Number P09962), the phage P2 TM protein Y (Swiss-Prot Accession Number P51773), the M protein of phage PRD1 (Swiss-Prot Accession Number P27389), the holin protein of phage A118 (Swiss-Prot Accession Number Q37975), the phage A500 holin protein (Swiss-Prot Accession Number Q37977), the DPH holin protein of phage Dp-1 (Swiss-Prot Accession Number 003978), the holin protein of phage HP1 (Swiss-Prot Accession Number P51727), the holin protein of phage rlt (Swiss-Prot Accession Number Q38134), the lysis protein 17.5 of phages T7 (Swiss-Prot Accession Number P03802) and T3 (Swiss-Prot Accession Number P10307), the NucE holin protein of phage P2 Ogr (Swiss-Prot Accession Number Q54418), the holin protein isolated from *Haemophilus somnus* (Swiss-Prot Accession Number Q48281), the P10 protein of phage Phi-6 (Swiss-Prot Accession Number P11127), the protein rV of phage T4 (Swiss-Prot Accession Number P06808), the *Staphylococcus* phage Phi-11 holin (Swiss-Prot Accession Number Q38020), and the *Lactococcus* phage Tuc2009 holin (Swiss-Prot Accession Number Q38613).

Most preferably they are selected from the group having the SEQ ID No. 7, SEQ ID No. 8 and SEQ ID No. 9

Included are also "analoga" of the holin proteins. Analoga that substantially correspond to holins are those polypeptides with conservative changes or in which one or more amino acids of the amino acid sequence of the holin has been replaced with another amino acid, deleted and/or inserted, provided that the resulting protein exhibits substantially the same or even higher biological activity as the holin to which it corresponds.

"Conservative" changes are those changes which would not be expected to change the activity, charge or configuration of the protein and thus would not be expected to change the biological properties thereof. Conservative substitutions include an "analog" wherein at least one amino acid residue in the polypeptide has been conservatively replaced by a different amino acid. Suitable substitutions may be determined by routine experimentation to provide modified structural and functional properties of a synthesized polypeptide while maintaining the biological activity of the holin protein. One or more substitutions could preferably be created in accordance with the following list:
Ala (Gly, Ser); Arg (Lys); Asn (Gln, His); Asp (Glu); Cys (Ser); Gin (Asn); Glu (Asp); Gly (Ala, Pro); His (Asn, Gln); Ile (Leu, Val); Leu (Ile, Val); Lys (Arg, Gln, Glu); Met (Leu, Tyr, Ile); Phe (Met, Leu, Tyr), Ser (Thr); Thr (Ser); Trp (Thr); Tyr (Trp, Phe); and Val (Ile, Leu)

At the genetic level these analogs are generally prepared by site-directed mutagenesis of nucleotides in the DNA encoding the holin protein as described in e.g. Sambrook et al., Molecular Cloning: A Laboratory Manuel, CSH Laboratory, Cold Spring Harbor, N.Y., 1989.

Double stranded DNA (dsDNA) bacteriophages, such as phage Lambda, use a dual system for host cell lysis consisting of (i) a muralytic enzyme or endolysin with enzymatic activities against the glycosidic, amide, or peptide bonds of the peptidoglycan and (ii) a pore-forming holin that allows the endolysin that lacks any secretory signal sequences and accumulates in the cytoplasm to gain access to the peptidoglycan in a timed manner, eventually resulting in death of the cell [28, 29]. The holin function is non-specific for the endolysin since artificial lysis systems consisting of a holin and an endolysin originating from distinct bacteriophages have been shown to be lytically competent [29]. This also demonstrates the functional homology of the different holin proteins having distinct amino acid sequences and thus showing no homology on the DNA or amino acid level. Although holins encoded by dsDNA bacteriophages are divergent, they generally exhibit common features: (i) small sizes (60-145 amino acids), (ii) two, three, or four transmembrane-spanning domains, (iii) a hydrophilic N-terminus, and (iv) a highly polar, charge-rich C-terminal domain. The N-and C-terminal hydrophilic domains are known to function in the timing of the holin-mediated channel formation [30, 31]. Despite these common features, holins of dsDNA bacteriophages can be grouped into two general classes. Class I holins, of which S of bacteriophage λ (Lambda) (S^{λ}) is the prototype, are in general of a size of 95 amino acid residues or longer and harbor three potential membrane-spanning domains. Class II holins, such as S²¹ of phage 21 are usually smaller, ranging in size between 65 and 95 amino acid residues, and contain only two transmembrane-spanning domains.

Holins of dsDNA bacteriophages form oligomeric complexes [32, 33]. Only few holin molecules are necessary to lyse a bacterial cell. For S^{λ} it has been shown that holin is lethal for an *E*. *coli* cell at concentrations of 1-3 x 10³ molecules per cell [34]. Usually, a lysogenic bacterial culture undergoes lysis within minutes after onset of lysis. This precise temporal regulation likely involves the energy state of the membrane since adding an energy poison such as cyanide or dinitrophenol sufficiently late in the infective cycle can instantly trigger premature lysis of cells infected with bacteriophage λ or other bacteriophages [29]. For bacteriophage λ, the exact timing of host cell lysis is controlled by the molar ratio between the actual holin and a holin inhibitor (usually about 2:1) [35]. Both proteins are encoded by the same open reading frame (ORF). The actual holin (S105) originates from translational initiation at codon number 3. The holin inhibitor (S107) is only two amino acid residues longer and is a product of translational initiation at codon number 1. This dual-start motif (*i*.*e*. two in-frame ATG start codons separated by one or two codons of which at least one is positively charged [35] is not only found in the S^{λ} ORF but also in a number of other holin genes. The presence of a holin/holin-inhibitor pair has not only experimentally been demonstrated for S^{λ} but also for other phage-encoded lysis systems [30, 36, 37], which are incorporated herein by reference.

In terms of bacterial cell lysis, in the most attractive model [38], holins exist in two conformational states in the cytoplasmic membrane: a "pre-hole" state in which the holin accumulates in the membrane, and the "hole" state in which the actual hole is formed. As the number of holin proteins in the membrane increases, holin dimers accumulate which are the building materials for higher-order oligomers, which eventually fuse to small holin islands. These holin arrays represent instable regions in the membrane and it is thought, that at some point, thermal fluctuation opens a hole within the holin arrays leading to a local depolarization of the membrane. This local depolarization then triggers the conformational change of the surrounding holin proteins from the pre-hole state to the hole state resulting in larger holes and a complete collapse of the membrane potential. In a chain reaction, the breakdown of the membrane potential would trigger the conformational change of more and more holin proteins into the active conformation, eventually leading to the creation of large holes in the cytoplasmic membrane. The hole itself has been shown to be of irregular size but must at least be large enough to allow the endolysin to pass through. Wang *et al*. have shown that the S^{λ} hole in the cytoplasmic membrane of *E*. *coli* is even large enough to allow the release of a 480 kDa endolysin/LacZ fusion protein [39].

In contrast to dsDNA bacteriophages, simple lytic bacteriophages such as the ssDNA bacteriophages (represented by φX174, α3, G4, S13) and the ssRNA bacteriophages (represented by MS2, GA, Qβ, SP) harbor only a single lysis gene coding for a small integral membrane protein. The lysates of these bacteriophages have been shown to be free of any muralytic enzyme activity and the amino acid sequences of the known holins from these bacteriophages do not indicate such a function either. Expression of plasmid-encoded φX174 lysis gene *E* in *E*. *coli* alone is sufficient for efficient cell lysis [40-42]. However, in this case, cell lysis is dependent on actively growing cells [43, 44]. Furthermore, it has been shown, that the bacterial host gene *slyD* coding for a peptidyl-prolyl cis-trans isomerase (PPlase) is absolutely required for cell lysis by wild type protein E [45]. SlyD is probably involved in the correct folding and stabilization of protein E or its delivery to the cytoplasmic membrane [46].

The present invention describes the toxic effect of the bacteriophage holin proteins, exemplarily using the phage λ-encoded *S105* protein, when expressed in animal cells, in particular in human cells, e.g. a human cervix carcinoma cell line (HeLa), a mammary adenocarcinoma cell line (MCF7), a human embryonic kidney cell line (HEK 293) and a murine mammary adenocarcinoma cells (TS/A) [72]. Thus, the present invention shows the cytotoxic effect of *S105* by describing the morphological changes of the cells after induction of *S105* expression, the cytotoxic effect on membrane integrity (*i*.*e*. by trypan blue staining, annexinV-PE/7AAD staining), and the cytotoxic effect on cellular metabolism (*i*.*e*. measuring NADPH or NADH levels generated by dehydrogenases or the membrane potential of mitochondria). By means of differential cell fractionation, fluorescence immunocytochemistry and transmission electron microscopy it is demonstrated that the Lambda *S105* protein accumulates preferably in mitochondria and the endoplasmic reticulum (ER) which leads to an disintegration of the mitochondrial ultrastructure and induction of cell death. It was shown that the expression of bacteriophage holin proteins induces a cell growth inhibitory activity and cell killing effect in animal, especially human cells *in-vitro* but also *in-vivo* as revealed in animal studies using tumor cell derived xenografts. This cell growth inhibitory activity and cell killing effect is particularly useful for limitating the growth activity of cancer cells or for eradicating cancer cells in a patient and thus for the treatment of cancer and other proliferative diseases.

For the use according to the present invention the bacteriophage-derived proteins, e.g. holins, need to be expressed in an eukaryotic, especially an animal or a human cell. For this purpose the nucleic acid sequence, e.g. DNA, cDNA, RNA of the phage-derived proteins or functional fragments thereof, or the expression product thereof, needs to be transferred into the cell. Alternatively, the nucleic acid sequence, e.g. RNA or mRNA encoding the holins, needs to be directly synthesised within the cell. In another approach the bacteriophage-derived protein or a fusion protein consisting of a bacteriophage-derived protein or a biological active portion thereof and a shuttle protein could be transferred to the target cell.

A "nucleic acid sequence" according to the present invention includes any nucleic acid sequence - e.g. DNA, cDNA, RNA, mRNA -, which encodes for bacteriophage-derived proteins or polypeptides having a holin-like function, e.g. holins, or biological active portions thereof. This definition embraces also nucleic acid sequences encoding the same polypeptide or protein but differing from the natural sequence due to changes permitted by the known degeneracy of the genetic code. And this definition also includes those nucleic acids capable of hybridizing, under stringent hybridization conditions, to the claimed nucleic acid sequences, induing mRNA, cDNA, genomic DNA and functional fragments thereof. Especially a nucleic acid sequence encoding for a fusion protein consisting of a holin or a biological active portion thereof and a shuttle protein (e.g. HIV-1-Tat, HBV-PreS2-TLM, Sox10, VP22) are also included into the definition.

A "producer cell" according to the present invention is defined to be a recombinant cell that is modified to either express the bacteriophage-derived proteins e.g. holins, "analoga" thereof, functional fragments thereof or "fusion proteins" encoded by the "nucleic acid sequence".

As used herein, the term "stringent hybridization conditions" refers to conditions for hybridization and washing under which nucleotide sequences typically remain hybridized to each other. Such stringent conditions are known to those skilled in the art and can e.g. be found in Current Protocols in Molecular Biology, John Wiley & Sons, N.Y. (1989), 6.3.1.ff. A variety of modifications can be made to DNA and RNA and thus, the term "nucleic acid" also embraces chemically, enzymatically, or metabolically modified forms. It is self explanatory that a "nucleic acid" which hybridizes only to polyA+ sequences like any 3' terminal polyA+ tract of a cDNA, or to a complementary stretch of T (or U) residues, would not be included in the definition of "nucleic acid," since such a nucleic acid would hybridize to any nucleic acid molecule containing a poly (A) stretch or the complement thereof.

For the transfer of nucleic acid sequences into a cell several known vector systems, viral vectors or also naked DNA could be used. In general vectors according to the present invention are DNA vehicles of linearised or circular structure, such as plasmids, cosmids or artificial chromosomes, which in addition to the desired nucleic acid sequence contain regulatory sequences, optionally selective marker genes and optionally replicons enabling the autonomous replication of the vector.

Several methods are known and can be applied to transfer nucleic acid sequences into target cells for the generation of transiently or stably transfected cells. These methods include physical and chemical methods such as electroporation, microinjection, microprojectile bombardment, antibody- or ligand-, dextran-, liposomal-, virosomal-, calcium phosphate-, charged polymer- or charged lipid-mediated DNA transfer. Chemical modifications e.g. derivatisation of peptides, proteins and oligonucleotides with lipophilic structures, may also be used to enhance the uptake into target cells. For instance, the sequence of a known membranotropic peptide may be fused to the nucleic acid sequence as mentioned above, or chemically modifications known to a person skilled in the art could be made to the holins, such as adding lauroyl derivatives, or oxidation of methionine residues to create sulfoxide groups, or replacing the peptide bond by its ketomethylene isoesters to enhance the membrane permeability. Alternatively, molecules that are known to bind to cell surface receptors could be conjugated to such polypeptides, proteins or nucleic acid sequences. Useful examples are sugars, vitamins, hormones, cytokines, transferrin, asiaglycoprotein as described in US 5,108,921, incorporated herein by reference.

According to a preferred embodiment the present invention uses and provides viral vector systems for the transduction of the coding information of the phage-derived holins or biologically active fragments thereof into the eukaryotic cell. In general every viral vector system, which allow the transfer and the subsequent expression of a nucleic acid sequence can be used according to the present invention. Particularly useful are poxviral expression vectors, adenoviral expression vectors, herpes virus expression systems, adeno-associated virus vectors, alphavirus vectors, SV40 vectors, vesicular stomatitis virus vectors, measles virus vectors, sindbis virus vectors, rabies virus vectors, as well as lentiviral and retroviral vector systems.

In a further preferred embodiment retroviral vector systems are used. Such retroviral vector systems are based either on replication competent (e.g. as described in WO 018240) or on replication deficient retroviral vector systems. While a replication competent retroviral vector system has the advantage of a high transduction rate, it also bears the risk of unspecific transduction. Specificity of gene expression needs therefore to be controlled by specific regulatory elements, such as cell-specific promoters.

In comparison to this, replication deficient retroviral vector systems, like the promoter conversion vector (ProCon) as described in EP 0779929 or a reconstituting viral (ReCon) vector as described in WO 99/35280 (both incorporated herein by reference), show high cell specificity but are less effectively transducing cells. Since various retroviral vector systems are well described in the art, it is easy for a person skilled in the art to choose a suitable vector system.

In general retroviral vector systems consist of two components, whereby the first component, the retroviral vector itself is a modified retrovirus (vector plasmid) in which the genes encoding for the viral proteins have been replaced by the nucleic acid sequence of interest optionally including marker genes to be transferred to the eukaryotic cell. Since the replacement of the genes coding for the viral proteins effectively cripples the virus, it must be rescued by the second component of the system, which encodes the missing viral proteins. The second component is a cell line that produces large quantities of the viral structural and enzymatic proteins, however lacks the ability to produce replication competent virus. This cell line is known as the packaging cell line and consists of a cell line transiently or stably transfected with one or more plasmids carrying the genes enabling the modified retroviral vector to be packaged. These plasmids direct the synthesis of the necessary viral proteins required for virion production. To generate viral particles or the packaged vectors, the first component or the vector plasmid is transferred into the packaging cell line. Under these conditions the modified retroviral genome induing the inserted therapeutic and optional marker genes is transcribed from the vector plasmid and packaged into the modified retroviral particles (recombinant viral particles). A cell infected with such a recombinant viral particle cannot produce new vector virus since no viral proteins are present in these cells. However, the vector carrying the therapeutic and marker genes is present and these can now be expressed in the infected cell.

In another preferred embodiment, a reconstituting retroviral vector, as described in WO 99/35280 could be used. In this vector system genetic reshuffling during the viral life cycle is exploited, allowing reconstitution of functional expression cassettes from separated elements exclusively in transduced target cells [52]. For this purpose, a heterologous regulatory element or promoter is inserted in the inverse orientation into the unique 5' region (U5) of the long terminal repeat (5' LTR) of the retroviral vector. The therapeutic toxic gene is inserted in the reverse orientation into the unique 3' region (U3) of the 3' long terminal repeat (3'LTR). After reverse transcription, both unique regions are duplicated, reconstituting two functional and active expression cassettes within each of the retroviral LTRs. The use of tissue-specific promoters may ensure expression of the transduced gene only or preferentially in the respective target cells or cells of specific tissues. All promoters can additionally be either constitutive or conditionally active and of viral or non-viral origin.

As used herein, the term "regulatory element" means a nucleic acid sequence that serves as a promoter, i.e., regulates expression of a nucleic acid sequence operably linked to the promoter. Such "regulatory elements" or "promoters" can control the expression of linked nucleic acid sequences either constitutively or inducible. Examples for inducible promoters are tetracycline-dependent regulatory systems (for review see [66]), heat shock, hormone (e.g. ecdysone, doxycyline, rifampicin), or metal ion regulatory systems. Many systems exhibit a significant level of basal expression. This "leakiness" makes it difficult to use them for the expression of cytotoxic genes. "Leaky promoters" according to the present invention are inducible regulatory elements, which exhibit a basal expression of the linked nucleic acid, without induction. The term "tissue-specific promoter" (TSP) means a regulatory element, which is only or primarily active in specific cells or specific cells of a tissue (e.g probasin promoter, human telomerase reverse transcriptase promoter, human surfactant protein A1 promoter, Ksp-cadherin (Cadherin 16) promoter, mouse mammary tumour virus promoter (MMTV-LTR), whey acidic protein promoter (WAP)).

In another preferred embodiment the promoter conversion vector (ProCon) as described in EP 0779929 could be used. According to the ProCon principle, a retroviral vector is constructed in which the 3' U3 region is altered, but the normal 5' U3 structure is maintained; the vector can be normally transcribed into RNA utilizing the normal retroviral promoter located within the 5' U3 region. However the generated RNA will only contain the altered 3' U3 structure. In the infected target cell, after reverse transcription, this altered U3 structure will be placed at both ends of the retroviral structure.

If the altered region carries e.g. a polylinker instead of the U3 region then any promoter, including those directing tissue-specific expression, can be easily inserted. This promoter will then be utilized exclusively in the target cell for expression of linked genes carried by the retroviral vector. If it is desired the promoter can be the only promoter in the vector. Additional DNA segments homologous to one or more celluar sequences can be inserted into the polylinker for the purposes of gene targeting.

In the packaging cell line the expression of the retroviral vector is regulated by the normal unselectively active retroviral promoter. However as soon as the vector enters the target cell, promoter conversion occurs and the therapeutic genes are expressed from a tissue-specific promoter of choice introduced into the polylinker. Not only can virtually any tissue-specific promoter be included in the system, providing for the selective targeting of a wide variety of different cell types, but additionally, following the conversion event, the structure and properties of the retroviral vector no longer resembles that of a virus. This, of course, has extremely important consequences from a safety point of view, since ordinary or state of the art retroviral vectors readily undergo genetic recombination with the packaging vector to produce potentially pathogenic viruses. Promoter conversion (ProCon) vectors do not resemble retroviruses because they no longer carry U3 retroviral promoters after conversion thus reducing the possibility of genetic recombination. Also ProCon vectors, in contrast to ordinary state of the art vectors, may be less likely to activate cellular genes in the vicinity of the integration sites.

In the content of the present invention and to allow the evaluation of a potential toxic effect of the expressed gene a tightly controlled gene expression system is used. A tight control of expression of the toxic gene is particularly important when using viral vector systems, wherein the viral vector needs to be packaged by a packaging cell and, of course, the expression of the toxic gene in the packaging cell would kill said cell. Only after the viral vector has been packaged into a viral particle, and only after the viral particle has infected its host, preferably a eukaryotic cell, more preferably an animal cell, particularly a mammalian or human cell, the toxic gene should be expressed in the host cell.

This could be achieved by e.g. using tight controlled inducible promoters operably linked to the cytotoxic gene. In a tightly controlled system the gene expression can be induced by adding the inducer. Without the inducer the system will be silent and no gene expression occurs. Accordingly, by adding the inducer only after the viral particle has infected the host cell it can be secured that expression of the toxic gene only occurs in the host cell. Examples for inducible promoters are the lac repressor/operator, FK506/rapamycin, ecdysone-inducible, RU486/mifepristone, and tetracycline (Tc)-inducible systems [68, 69] or the mouse mammary tumor virus promoter (MMTV-LTR).

The present invention, in a further embodiment, identifies the value of λ-*S105*, λ-*S107,* and Φ*29-GP14* as toxic proteins for cancer gene therapy using (i) a hormone-inducible gene expression system based on retroviral vectors using the mouse mammary tumor virus (MMTV) promoter that can be activated by glucocorticoids [65] and (ii) tetracycline/doxycycline inducible gene expression system based on two different plasmid vectors [67, 68]. Using these inducible gene expression systems for induction a significant effect of the *S105* expression could be exemplarily demonstrated on the morphology, on membrane integrity (*i*.*e*. by trypan blue staining, annexinV-PE/7AAD staining), and on cellular metabolism (*i*.*e*. measuring NADPH or NADH levels generated by dehydrogenases or measuring membrane potentials) either of HeLa cells, MCF-7 cells or HEK 293 cells (see Examples). It was further shown by means of fluorescence immunocytochemistry and differential cell fractionation that λ-*S105,* λ-*S107,* and Φ*29-GP14* when expressed within a cell accumulate preferably in the mitochondria as well as in the endoplasmic reticulum of the cell which results in a rapid destruction of the mitochondrial membrane potential. Using animal xenograft models it was demonstrated that the expression of bacteriophage-derived proteins resulted in significantly reduced growth rates of the tumours, compared to a non-treated control group not expressing the therapeutic bacteriophage-derived holin. Thus, it was unexpectedly shown that the expression of bacteriophage-derived holins in human cells leads to a reduction in cell growth and also specifically kills eukaryotic cells, especially animal cells or more specifically mammalian cells including human cells *in vitro* as well as *in-vivo*.

According to another embodiment also conventional regulatory elements could be used. Basal transcription from leaky regulatory elements could be silenced by expressing within the packaging system a nucleic acid sequence at least partially complementary to the transcript of the cytotoxic gene (antisense RNA) but still capable of efficiently binding to the transcript of the cytotoxic gene and therefore inhibiting translation of the cytotoxic gene in a way that at least the vitality of the packaging system is prolonged and sufficient viral vectors particles are produced.

The invention - according to a further embodiment - provides an in vitro method to introduce and express the bacteriophage-derived protein or fusion protein, which induces a cell growth inhibitory activity or cell killing effect, or at least the functional part thereof in a eukaryotic cell. For this the nucleic acid sequence encoding the bacteriophage-derived protein or the fusion protein, the functional part thereof or analoga thereof and an operably linked regulatory element is introduced into the eukaryotic cell, where it subsequently will be expressed under the control of a suitably linked regulatory element. For uptake said nucleic acid sequence can be presented as naked DNA to the eukaryotic cell, which will to a certain extent take up the nucleic acid sequence and express the encoded protein. To increase the efficacy plasmids or other DNA vectors could be transfected into the cells using well-known transfection systems, such as e.g. calcium phosphate. Viral vectors, which could be either recombinant viruses or viral particles, would be used to infect the eukaryotic cell and would thereby transfer the nucleic acid sequence into said cells.

A "fusion protein" according to the present invention consists of a first and at least a second polypeptide, all of them operatively linked to each other whereas each polypeptide preserves its specific function.

In embodiments in which the fusion protein is designed to enter a target cell from the extracellular medium e.g. released from compositions or capsules, a bacteriophage-derived protein can be linked to a protein transduction domain (PTD) or in other words shuttle protein, which ease take up by a cell. Examples for such transduction domains are VP22 of Herpes simplex virus, PTD domain of Antennapedia protein, PTD of PDX-1 or the protein transduction domain of the Human immunodeficiency virus protein Tat.

In a preferred embodiment the 11-amino acid residues that constitute the protein transduction domain of the HIV Tat protein is linked to the NH₂-terminal either of a bacteriophage-derived protein or of a holin, or preferably of phage Lambda *S105* protein, phage Lambda *S107* protein, or phage Phi29 *GP14* protein.

In embodiments in which the fusion proteins are secreted from a producer cell into the extracellular medium the polynucleotide sequence that encodes either the bacteriophage-derived protein or a fusion protein consisting of a protein transduction domain and a bacteriophage-derived holin is linked to a polynucleotide sequence that encodes a cleavable signal peptide sequence. The signal sequence of the fusion protein directs translocation of the fusion protein through the cell membrane. Some naturally occurring signal peptides of different proteins are not homologous in sequence, but they generally include several, preferably 4 to 12 hydrophobic residues, which are essential for crossing the endoplasmic reticulum and thus allowing the protein to be transported into golgi apparatus or exocytotic release bodies. Generally, a basic residue is additionally located a few residues before the hydrophobic sequence of the signal peptide.

In a preferred embodiment of the invention the signal peptide is operatively linked to the N-terminus of the bacteriophage-derived protein, more preferably to a fusion protein consisting of a protein transduction domain and a bacteriophage-derived holin, and further preferably the protein transduction domain consists of the 11-amino acid residues of HIV Tat and the holin is chosen from either phage Lambda *S105,* phage Lambda *S107* or Phi29 *GP14*. Examples of cleavable signal peptides that could be used according to the present invention could be chosen e.g. from Preproalbumin, Pre-IgG light chain, Prelysozyme, Preprolactin, Prepenicillinase, Prevesicular stomatitis virus glycoprotein, or Prelipoprotein. Further preferably the osteonectin/SPARC/BM40 signal peptide is used.

The "fusion protein" according to the present invention can be produced by methods already known in the art. These methods include, for example, *in vitro* recombinant DNA techniques, chemical techniques and *in vivo* recombination/genetic recombination. DNA and RNA synthesis may, additionally, be performed using an automated synthesizer as described e.g. in Sambrook *et al*., 1989).

The preparation of such a fusion protein generally entails the preparation of a first and second DNA coding region and the functional ligation or joining of such regions, in frame, to prepare a single coding region that encodes the desired fusion protein. In the present context, a DNA sequence coding for the bacteriophage-derived protein, preferably a holin, further preferably a Lambda *S105* protein is joined in frame with a DNA sequence encoding a shuttle protein (e.g. HIV-1-Tat, HBV-PreS2-TLM, Sox10 or VP22). It is not generally believed to be particularly relevant which portion of the therapeutic agent-targeting agent is prepared as the N-terminal region or as the C-terminal region. Depending on the shuttle protein used as part of the fusion protein, it may be necessary to provide a peptide spacer for linkage of the two parts of the fusion protein. These peptide spacers could be either cleavable or non-cleavable. Such recombinantly produced fusion proteins may be purified and formulated for human administration. Alternatively, nucleic acids encoding the fusion proteins may be delivered via gene therapy. Although naked recombinant DNA or plasmids may be employed, the use of liposomes or vectors is preferred.

Alternatively, cross-linking reagents could be used to form molecular bridges that chemically tie together functional groups of two different molecules. To link two different proteins in a step-wise manner, hetero-bifunctional cross-linkers are preferred that eliminate unwanted homopolymer formation. Hetero-bifunctional cross-linkers contain two reactive groups: one generally reacting with primary amine group (e.g., N-hydroxy succinimide (NHS)) and the other generally reacting with a thiol group (e.g., pyridyl disulfide, maleimides, halogens, etc.). Through the primary amine reactive group, the cross-linker may react with the lysine residue(s) of one protein and through the thiol reactive group, the cross-linker, already tied up to the first protein, reacts with the cysteine residue (free sulfhydryl group) of the other protein.

Therefore, polypetides or proteins generally have, or are derivatized to have, a functional group available for cross-linking purposes. This requirement is not considered to be limiting in that a wide variety of groups can be used in this manner. For example, primary or secondary amine groups, hydrazide or hydrazine groups, carboxyl alcohol, phosphate, or alkylating groups may be used for binding or cross-linking.

The spacer arm between the two reactive groups of cross-linkers may have various length and chemical compositions. A longer spacer arm allows a better flexibility of the conjugate components while some particular components in the bridge (e.g., benzene group) may lend extra stability to the reactive group or an increased resistance of the chemical link to the action of various aspects (e.g., disulfide bond resistant to reducing agents). The use of peptide spacers, such as L-Leu-L-Ala-L-Leu-L-Ala, is also contemplated.

It is preferred that a cross-linker having reasonable stability in blood or other body fluids will be employed. Numerous types of disulfide-bond containing linkers are known that can be successfully employed. Exemplary hetero-bifunctional cross-linkers are SMPT, SPDP, LC-SPDP, Sulfo-LC-SPDP, SMCC, Sulfo-SMCC, MBS, Sulfo-MBS, SIAB, Sulfo-SIAB, SMPB, Sulfo-SMPB, EDC/Sulfo-NHS or ABH. Linkers that contain a disulfide bond that is sterically hindered may prove to give greater stability *in vivo,* preventing release of the agent prior to binding at the site of action. These linkers are thus one preferred group of linking agents.

One of the further preferred cross-linking reagents used is SMPT, which is a bifunctional cross-linker containing a disulfide bond that is "sterically hindered" by an adjacent benzene ring and methyl groups. It is believed that steric hindrance of the disulfide bond serves a function of protecting the bond from attack by thiolate anions such as glutathione which can be present in tissues and blood, and thereby help in preventing decoupling of the conjugate prior to the delivery of the attached agent to the target site e.g. a tumour.

The SMPT cross-linking reagent, as with many other known cross-linking reagents, lends the ability to cross-link functional groups such as the SH of cysteine or primary amines (e.g., the epsilon amino group of lysine). Another possible type of cross-linker includes the hetero-bifunctional photoreactive phenylazides containing a cleavable disulfide bond such as sulfosuccinimidyl-2-(p-azido salicylamido) ethyl-1,3'-dithiopropionate. The N-hydroxy-succinimidyl group reacts with primary amino groups and the phenylazide (upon photolysis) reacts non-selectively with any amino acid residue.

In addition to hindered cross-linkers, non-hindered linkers can also be employed in accordance herewith. Other useful cross-linkers, not considered to contain or generate a protected disulfide, include SATA, SPDP and 2-iminothiolane. The use of such cross-linkers is well understood in the art.

Once conjugated, the conjugate is separated from unconjugated components and from other contaminants. A large a number of purification techniques are available for use in providing conjugates of a sufficient degree of purity to render them clinically useful. Purification methods based upon size separation, such as gel filtration, gel permeation or high performance liquid chromatography, will generally be of most use. Other chromatographic techniques, such as Blue-Sepharose separation, may also be used.

A "pharmaceutical composition" according to the invention may include beside a therapeutically effective amount of the expression vector, the nucleic acid sequence, the viral particle, the eukaryotic packaging cell, the producer cell, the bacteriophage-derived protein e.g. holing and/or the fusion protein, in general, one or more pharmaceutical acceptable and/or pharmacologically acceptable carriers, additives, antibiotics, preservatives, adjuvants, diluents and/or stabilizers.

The phrases "pharmaceutically or pharmacologically acceptable" refer to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. Further such auxiliary substances can be water, saline, glycerol, ethanol, wetting or emulsifying agents, pH buffering substances, or the like. Suitable carriers are typically large, slowly metabolized molecules such as proteins, polysaccharides, polylactic acids, polyglycolic acids, polymeric amino acids, amino acid copolymers, lipid aggregates, or the like. The use of such media and agents for pharmaceutical active substances is well known in the art. For human administration, preparations should meet sterility, pyrogenicity, general safety and purity standards as required by FDA Office of Biologics standards or according to the EudraLex rules governing medicinal products in the European Union.

In another embodiment a therapeutically effective amount of expression vector, the nucleic acid sequence, the viral particle, the eukaryotic producer or packaging cell and/or the bacteriophage-derived protein or fusion protein could be encapsulated into permeable or semipermeable membranes.

The term "encapsulated" means that the biomaterials are contained in a biocompatible device or "capsule" which immuno-isolates the content from the surrounding environment and allows the release of the expression vector, the nucleic acid sequence, the viral particle and/or the bacteriophage-derived protein e.g. a holin out of the capsules. The main characteristics of the biocompatible material used for encapsulation should therefore be as follows: (i) it does not significantly activate the complement system; (ii) allows the passage of smaller molecules such as oxygen, nutrients and waste products in and/or out of the capsules; (iii) it allows the release of the product (expression vector, the nucleic acid sequence, the viral particle and/or the bacteriophage-derived protein e.g. a holin out of the capsules; and (iv) it significantly prevents the entry of components of the host immune system into the capsules.

Various biocompatible artificial polymers (or copolymers) can be employed for encapsulation, e.g. derived from cellulose, dextran, polyamide, polyurethane, acrylonitril, nylon, alginate-poly-L-lysine, hydroxyethyl methacrylate (HEMA), AN69 (polyacrylonitril-methallylsulfonate), hydroxyethyl methacrylate-methyl methacrylate (HEMA-MMA), polyamides, polyethersulfones (PES), chitosan sulfate, PAN/PVC, or agarose-polystyrene alginate. Preferably the semipermeable membrane is formed by electrolyte complexes, most preferably, cellulose sulfate and poly-dimethyldiallylammonium chloride.

The biocompatible device can exhibit different forms e.g. hollow fibers, sheets or cushions. In a particular embodiment of this invention, the capsule is a sphere and could have a variable diameter between 0.01 and 5 mm, preferably between 0.1 and 2 mm. As an example microcapsules as described in EP0835137 or EP0892852 and incorporated herein by reference could be used.

For encapsidation the expression vector, the nucleic acid sequence, the viral particle, the producer cell, the packaging cell and/or the bacteriophage-derived protein e.g. the holin is mixed with the poly-anionic component (e.g. cellulose sulphate) in an appropriate medium. The mixture is then dropped into a polymerisation bath containing the poly-cationic component (e.g. polydiallyldimethylammonium chloride, polyvinylbenzyltrimethylammonium chloride) resulting in capsules exhibiting a stabile membrane formed by the electrolyte complex and surrounding a fluid core of the unpolymerized poly-anion.

For preparation of such microcapsules several production methods are known (e.g. AirJet-method, Vibration-method, JetCutter-method).

After formation of the capsule the biomaterial is entrapped within the capsule core.
For preparation of a pharmaceutical composition it is necessary to separate the capsules from the polymerisation medium using additional washing and/or filtration steps.

The composition containing the capsules is preferably formulated for parenteral administration, e.g. for injection via the intravenous, intraarterial, intramuscular, or other such routes, including direct instillation into a tumor or disease site (intracavity administration) of an animal or a human in need thereof.

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. Media fulfilling the requirements for *in vivo* use in humans are e.g. NaCl solutions or Rin ger-Lactate solutions. In all cases, the form should be sterile and fluid to the extent that syringability exists. It should be stable under the conditions of manufacture and storage and should be preserved against the contaminating action of microorganisms, such as bacteria and fungi. Capsules can be formulated in suitable containers like glass vials or syringes. Formulations containing encapsulated cells may further contain a cryo-protectant (e.g. DMSO, Glycerol) and/or further additives. The concentration of cryo-protectants may be reduced by either dilution or additional washing steps prior to application to the patient.

Furthermore, according to further embodiments of the present invention the bacteriophage-derived protein, the holing or the fusion protein could be combined in a kit with other pharmaceutically acceptable formulations. Such other formulations could be, either for diagnosis/imaging or combined therapy. For example, such kits may contain any one or more of a range of chemotherapeutic or radiotherapeutic drugs; anti-angiogenic agents; anti-tumor cell antibodies; and/or anti-tumor vasculature or cytotoxins.

The bacteriophage-derived proteins or fusion proteins of the present invention will most likely be formulated for parenteral administration, e.g., formulated for injection via the intravenous, intramuscular, sub-cutaneous, transdermal, or other such routes, including peristaltic administration and direct instillation into a tumor or disease site (intracavity administration).

The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form should be sterile and fluid to the extent that syringability exists. It should be stable under the conditions of manufacture and storage and should be preserved against the contaminating action of microorganisms, such as bacteria and fungi.

The pharmaceutical compositions can be formulated into a sterile aqueous composition in a neutral or salt form. Solutions of the therapeutic agent-targeting agents as free base or pharmacologically acceptable salts can be prepared in water suitably mixed with a surfactant, such as hydroxypropylcellulose. Pharmaceutically acceptable salts, include the acid addition salts (formed with the free amino groups of the protein), and those that are formed with inorganic acids such as, for example, hydrochloric or phosphoric acids, or such organic acids as acetic, trifluoroacetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

Suitable carriers include solvents and dispersion media containing, for example, water, ethanol, polyol (for example, glycerol, propylene glycol, and liquid polyethylene glycol, and the like), suitable mixtures thereof, and vegetable oils. In many cases, it will be preferable to include isotonic agents, for example, sugars or sodium chloride. The proper fluidity can be maintained, for example, by the use of a coating, such as lecithin, by the maintenance of the required particle size in the case of dispersion and/or by the use of surfactants.

In further embodiments the bacteriophage-derived protein e.g. holin or fusion protein could be entrapped in liposomes and/or nanoparticles and/or other cladding bodies. The formation and use of liposomes is generally known to those of skill in the art. Generally, liposomes are formed from phospholipids that are dispersed in an aqueous medium and spontaneously form multilamellar concentric bilayer vesicles (also termed multilamellar vesicles (MLVs). MLVs generally have diameters of from 25 nm to 4 µm. Sonication of MLVs results in the formation of small unilamellar vesicles (SUVs) with diameters in the range of 200 to 500 angstrom, containing an aqueous solution in the core. Liposomes interact with cells via different mechanisms e.g. endocytosis by phagocytosis or fusion with the plasma cell membrane by insertion of the lipid bilayer of the liposome into the plasma membrane, with simultaneous release of liposomal contents into the cytoplasm of the target cell.

Nanocapsules or other cladding bodies according to this part of the invention can generally entrap compounds in a stable and reproducible way. To avoid side effects due to intracellular polymeric overloading, such capsules should be designed using polymers able to be preferably degraded *in vivo,* e.g. using biodegradable polyalkylcyanoacrylate.

A therapeutically effective amount of the bacteriophage-derived protein or fusion protein could be in the range of about 5-100 mgs, about 10-80 mgs, about 20-70 mgs, preferably about 25-60 mgs, or further preferably about 30-50 mgs per dose. It will be understood that lower doses may be more appropriate in combination with other agents, and that higher doses greater than 100 mgs can still be tolerated.

Within other aspects of the present invention, methods are provided for preparing a composition containing and preserving an infectious viral particle or recombinant virus. Briefly, infectious viral particles or recombinant viruses which have been purified or concentrated may be preserved by first adding a sufficient amount of a formulation buffer to the media containing the recombinant virus, in order to form an aqueous suspension. The formulation buffer is an aqueous solution that contains a polysaccharide, a high molecular weight structural additive, and a buffering component in water. As utilized within the context of the present invention, a "buffering compound" or "buffering component" should be understood to refer to a substance that functions to maintain the aqueous suspension at a desired pH. The aqueous solution may also contain one or more amino acids and/or optionally a cellular growth factor.

The infectious viral particles or recombinant viruses can also be preserved in a purified form. More specifically, prior to the addition of the formulation buffer, the crude stock may be clarified by passing it through a filter, and then concentrated, such as by a cross flow concentrating system (Filtron Technology Corp., Nortborough, MA). Within one embodiment, DNase is added to the concentrate to digest exogenous DNA. The digest is then diafiltrated to remove excess media components and establish the infectious viral particles or recombinant virus in a more desirable buffered solution. The diafiltrate is then passed over a Sephadex S-500 gel column and a purified particles or virus is eluted. A sufficient amount of formulation buffer is added to this eluate to reach a desired final concentration of the constituents and to minimally dilute the recombinant virus, and the aqueous suspension is then stored, preferably at -70°C or immediately dried. As noted above, the formulation buffer is an aqueous solution that contains a saccharide, a high molecular weight structural additive, and a buffering component in water. The aqueous solution may also contain one or more amino acids and/or optionally a cellular growth factor.

The crude stock of infectious viral particle or recombinant virus can also be purified by an ultracentrifugation step through a sucrose density gradient. This method is known as a standard procedure of virus purification. In general, the crude stock can be purified by making use of the fact that molecules of a particular density centrifuged in a solution of varying density (e.g. 20% - 60% w/v sucrose in 0.1 M Tris-EDTA pH 9.5) will tend to collect in a band at that zone where the density of the molecule and the density of the solution are exactly equal. After centrifugation, different fractions are collected through a hole in the bottom of the centrifugation tube. Superfluous sucrose is washed out by centrifugation in a buffered solution and the infectious viral particles or recombinant viruses are resuspended in a desired buffered solution. A sufficient amount of formulation buffer is then added, as discussed above, to the purified recombinant virus and the aqueous suspension is either dried immediately or stored, preferably at -70°C.

The aqueous suspension in crude or purified form can be dried by lyophilization or evaporation at ambient temperature. Specifically, lyophilization involves the steps of cooling the aqueous suspension below the glass transition temperature or below the eutectic point temperature of the aqueous suspension, and removing water from the cooled suspension by sublimation to form a lyophilized virus. Briefly, aliquots of the formulated recombinant virus are placed into an Edwards Refrigerated Chamber (3 shelf RC3S unit) attached to a freeze dryer (Supermodulyo 12K). A multistep freeze drying procedure as described by Phillips et al. (Cryobiology 18:414, 1981) is used to lyophilize the formulated recombinant virus, preferably from a temperature of -40°C to - 45°C. The resulting composition contains less than 10% water by weight of the lyophilized virus. Once lyophilized, the recombinant virus is stable and may be stored at -20°C to 25°C, as discussed in more detail below.

The aqueous solutions used for formulation, as previously described, are composed of a saccharide, high molecular weight structural additive, a buffering component, and water. The solution may also include one or more amino acids. The combination of these components act to preserve the activity of the infectious viral particle or recombinant virus upon freezing and lyophilization, or drying through evaporation. Although a preferred saccharide is lactose, other saccharides may be used, such as sucrose, mannitol, glucose, trehalose, inositol, fructose, maltose or galactose. In addition, combinations of saccharides can be used, for example, lactose and mannitol, or sucrose and mannitol. A particularly preferred concentration of lactose is 3% - 4% by weight. Preferably, the concentration of the saccharide ranges from 1% to 12% by weight.

The amino acids, if present, function to further preserve viral infectivity upon cooling and thawing of the aqueous suspension. In addition, amino acids function to further preserve viral infectivity during sublimation of the cooled aqueous suspension and while in the lyophilized state. A preferred amino acid is arginine, but other amino acids such as lysine, ornithine, serine, glycine, glutamine, asparagine, glutamic acid or aspartic acid can also be used. A particularly preferred arginine concentration is 0.1% by weight. Preferably, the amino acid concentration ranges from 0.1% to 10% by weight.

In addition, it may be advantageous to use aqueous solutions containing components known to enhance the activity of the cells to be infected. Growth factors or a combination of growth factors, if present in the pharmaceutical composition, are capable of regulating physiological processes of cells, and hence, in the context of the present invention facilitate the uptake and further integration of the recombinant retroviral vector. Representative examples include epidermal growth factor (EGF), melanocyte stimulating hormone (MSH), insulin like growth factor (IGF) and various cytokines, like Interleukin-2, -4 or-10, Interferon alpha or gamma.

The buffering component acts to buffer the solution by maintaining a relatively constant pH. A variety of buffers may be used, depending on the pH range desired, preferably between 7.0 and 7.8. Suitable buffers include phosphate buffer and citrate buffer. A particularly preferred pH of the recombinant virus formulation is 7.4, and a preferred buffer is tromethamine.

In addition, it is preferable that the aqueous solution contains a neutral salt which is used to adjust the final formulated virus to an appropriate iso-osmotic salt concentration. Suitable neutral salts include sodium chloride, potassium chloride or magnesium chloride. A preferred salt is sodium chloride.

Aqueous solutions containing the desired concentration of the components described above may be prepared as concentrated stock solutions.

A particularly preferred method of preserving recombinant viruses in a lyophilized state for subsequent reconstitution comprises the steps of (a) combining an infectious recombinant virus with an aqueous solution to form an aqueous suspension, the aqueous suspension including 4% by weight of lactose, 0.1% by weight of human serum albumin, 0.03% or less by weight of NaCl, 0.1% by weight of arginine, and an amount of tromethamine buffer effective to provide a pH of the aqueous suspension of approximately 7.4, thereby stabilizing the infectious recombinant virus; (b) cooling the suspension to a temperature of from -40°C to -45°C to form a frozen suspension; and (c) removing water from the frozen suspension by sublimation to form a lyophilized composition having less than 2% water by weight of the lyophilized composition, the composition being capable of infecting mammalian cells upon reconstitution. It is preferred that the recombinant virus is replication competent and further preferred that the recombinant virus is replication defective and suitable for administration into humans upon reconstitution.

It will be evident to those skilled in the art given the disclosure provided herein that it may be preferable to utilize certain saccharides within the aqueous solution when the lyophilized virus is intended for storage at room temperature. More specifically, it is preferable to utilize disaccharides, such as lactose or trehalose, particularly for storage at room temperature.

The lyophilized or dehydrated viruses of the subject invention may be reconstituted using a variety of substances, but are preferably reconstituted using water. In certain instances, dilute salt solutions, which bring the final formulation to isotonicity may also be used. In addition, it may be advantageous to use aqueous solutions containing components known to enhance the activity of the reconstituted virus. Such components include cytokines, such as IL-2, polycations, such as protamine sulfate, or other components, which enhance the transduction efficiency of the reconstituted virus. Lyophilized or dehydrated recombinant virus may be reconstituted with any convenient volume of water or the reconstituting agents noted above that allow substantial and preferably total solubilization of the lyophilized or dehydrated sample.

According to the present invention the compositions as described above are particularly useful for treating cancer in a patient in need thereof. For this, the compositions of the present invention may be administered directly to a tumor or to a wide variety of locations including, for example, into sites such as the cerebral spinal fluid, bone marrow, joints, arterial endothelial cells, rectum, buccal/sublingual, vagina, the lymph system, to an organ selected from the group consisting of lung, liver, pancreas, spleen, skin, blood and brain, or to a site selected from the group consisting of tumors and interstitial spaces. Within other embodiments, the composition may be administered intraocularly, intranasally, sublingually, orally, topically, intravesically, intrathecally, intravenously, intraarterially e.g. into the intrahepatic artery, intraperitoneally, intracranially, intramuscularly, intraarticularily or subcutaneously. Other representative routes of administration include gastroscopy, ECRP and colonoscopy, which do not require full operating procedures and hospitalization, but may require the presence of medical personnel.

Considerations for administering the compositions of the present invention include the following:
Oral administration is easy and convenient, economical (no sterility required), safe (over dosage can be treated in most cases), and permits controlled release of the active ingredient of the composition (the recombinant virus). Conversely, there may be local irritation such as nausea, vomiting or diarrhea, erratic absorption for poorly soluble drugs, and the recombinant virus will be subject to "first pass effect" by hepatic metabolism and gastric acid and enzymatic degradation. Further, there can be slow onset of action, efficient plasma levels may not be reached, a patient's cooperation is required, and food can affect absorption.
Buccal/sublingual administration is a convenient method of administration that provides rapid onset of action of the active component(s) of the composition, and avoids first pass metabolism. Thus, there is no gastric acid or enzymatic degradation, and the absorption of recombinant viruses is feasible. There is high bioavailability, and virtually immediate cessation of treatment is possible. Conversely, such administration is limited to relatively low dosages (typically about 10-15 mg), and there can be no simultaneous eating, drinking or swallowing.
Rectal administration provides a negligible first pass metabolism effect (there is a good blood/lymph vessel supply, and absorbed materials drain directly into the inferior vena cava), and the method is suitable for children, patients with emesis, and the unconscious. The method avoids gastric acid and enzymatic degradation, and the ionisation of a composition will not change because the rectal fluid has no buffer capacity (pH 6.8; charged compositions absorb best). Conversely, there may be slow, poor or erratic absorption, irritation, degradation by bacterial flora, and there is a small absorption surface (about 0.05 m²). Further, lipidophilic and water soluble compounds are preferred for absorption by the rectal mucosa, and absorption enhancers (e.g. salts, EDTA, NSAID) may be necessary.

Many of the routes of administration described herein (e.g., into the cerebrospinal fluid (CSF), into bone marrow, intravenous, intraarterial, intracranial, intramuscular, subcutaneous, into various organs, intratumoral, into the interstitial spaces, intraperitoneal, intralymphatic, or into a capillary bed) may be accomplished simply by direct administration using a needle, catheter or related device. In particular, within certain embodiments of the invention, one or more dosages may be administered directly in the indicated manner:
into the cerebral spinal fluid at dosages greater than or equal to 10⁵, 10⁶, 10⁷, 10⁸, 10⁹, 10¹⁰, or 10¹¹ cfu ; into bone marrow at dosages greater than or equal to 10⁵, 10⁶, 10⁷, 10⁸, 10⁹, 10¹⁰, or 10¹¹ cfu; intraarticularily at dosages greater than or equal to 10⁵, 10⁶, 10⁷, 10⁸, 10⁹, 10¹⁰, or 10¹¹ cfu; intravenously at dosages greater than or equal to 10⁸, 10⁹, 10¹⁰, or 10¹¹ cfu; intraarterially at dosages greater than or equal to 10⁵, 10⁶, 10⁷, 10⁸, 10⁹, 10¹⁰, or 10¹¹ cfu; intracranially at dosages greater than or equal to 10⁹, 10¹⁰, or 10¹¹ cfu; intramuscularly at dosages greater than or equal to 10¹⁰, or 10¹¹ cfu; intraocularily at dosages greater than or equal to 10⁵, 10⁶, 10⁷, 10⁸, 10⁹, 10¹⁰, or 10" cfu; pulmonarily at dosages greater than or equal to 10⁵, 10⁶, 10⁷, 10⁸, 10⁹, 10¹⁰, or 10¹¹ cfu; nasally at dosages greater than or equal to 10⁵, 10⁶, 10⁷, 10⁸, 10⁹, 10¹⁰, or 10¹¹ cfu; sublingually at dosages greater than or equal to 10⁵, 10⁶, 10⁷, 10⁸, 10⁹, 10¹⁰, or 10¹¹ cfu; rectally at dosages greater than or equal to 10⁵, 10⁶, 10⁷, 10⁸, 10⁹, 10¹⁰, or 10¹¹ cfu; orally at dosages greater than or equal to 10⁵, 10⁶, 10⁷, 10⁸, 10⁹, 10¹⁰, or 10¹¹ cfu; topically at dosages greater than or equal to 10⁵, 10⁶, 10⁷, 10⁸, 10⁹, 10¹¹, or 10¹¹ cfu; vaginally at dosages greater than or equal to 10⁵, 10⁶, 10⁷, 10⁸, 10⁹, 10¹⁰, or 10¹¹ cfu; subcutaneously at dosages greater than or equal to 10⁹, 10¹⁰, or 10¹¹ cfu; intervesically at dosages greater than or equal to 10⁵, 10⁶, 10⁷, 10⁸, 10⁹, 10¹⁰, or 10¹¹ cfu; into an organ such as the lung, liver, spleen, skin, blood or brain at dosages greater than or equal to 10⁵, 10⁶, 10⁷, 10⁸, 10⁹, 10¹⁰, or 10¹¹ cfu; intratumour at dosages greater than or equal to 10⁸, 10⁹, 10¹⁰, or 10¹¹ cfu; intraperitoneally at dosages greater than or equal to 10⁸, 10⁹, 10¹⁰, or 10¹¹ cfu; interstitial spaces at dosages greater than or equal to 10¹⁰ or 10¹¹ cfu; intralymphatically at dosages greater than or equal to 10⁵, 10⁶, 10⁷, 10⁸, 10⁹, 10¹⁰, or 10¹¹ cfu; into a capillary bed at dosages greater than or equal to 10⁵, 10⁶, 10⁷, 10⁸, 10⁹, 10¹⁰, or 10¹¹ cfu; or intrathecally at dosages greater than or equal to 10⁵, 10⁶, 10⁷, 10⁸, 10⁹, 10¹⁰, or 10¹¹ cfu.

Recombinant virus may be delivered to the target from outside of the body (as an outpatient procedure) or as a surgical procedure, where the vector is administered as part of a procedure with other purposes, or as a procedure designed expressly to administer the vector. Other routes and methods for administration include the non-parenteral routes as well as administration via multiple sites.

Alternatively, viruses, viral vector particles or packaging cells can be encapsulated into biocompatible devices e.g. capsulesas described above. Such biocompatible devices can be applied in form of compositions containing said biocompatible devices and formulated preferably for parenteral administration into an animal, including a human in need thereof.

### SHORT DESCRIPTION OF THE DRAWINGS

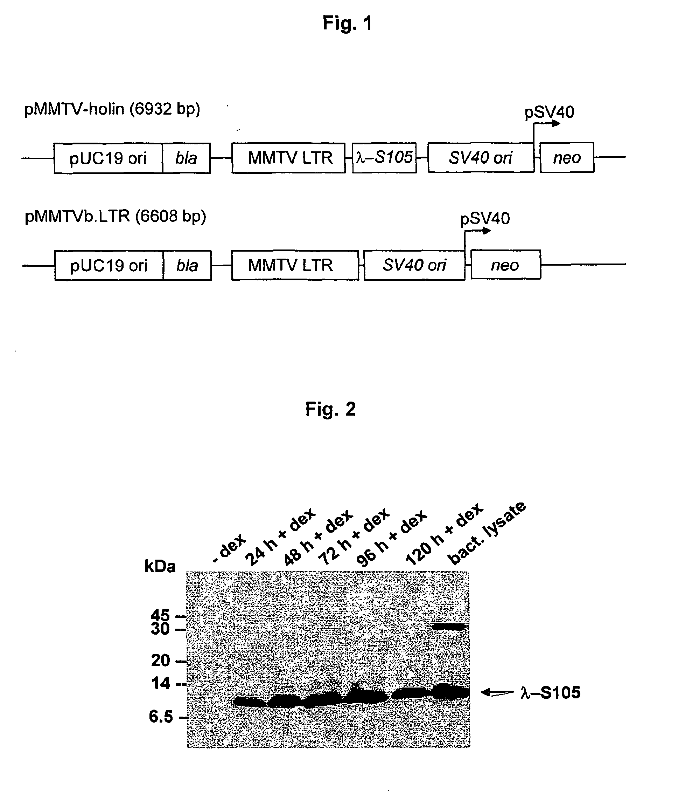

**Fig. 1** shows a schematic drawing of the expression vectors pMMTV-holin and pMMTVb.LTR: *bla*, β-lactamase gene; MMTV LTR, 3' LTR of Mouse Mammary Tumor Virus; λ-*S105*, bacteriophage λ gene S encoding the λ-holin protein (codons 3 to 107); SV40 ori, SV40 origin of replication; pSV40, SV40 promoter; neo, neomycin resistance gene; pUC19 ori, origin or replication from plasmid pUC19
**Fig. 2** shows the expression of λ-*S105* in HeLa cells stably transfected with pMMTV-holin after stimulation with dexamethasone for 24 h, 48 h, 72 h, 96 h and 120 h as determined by Western blotting. The stained λ-*S105* encoded holin protein is indicated by an arrow. The sizes of the molecular weight standard are given on the left (kDa); - dex, no dexamethasone treatment; bact. lysate, lysate of *E*. *coli* cells inducibly expressing λ-*S105*.

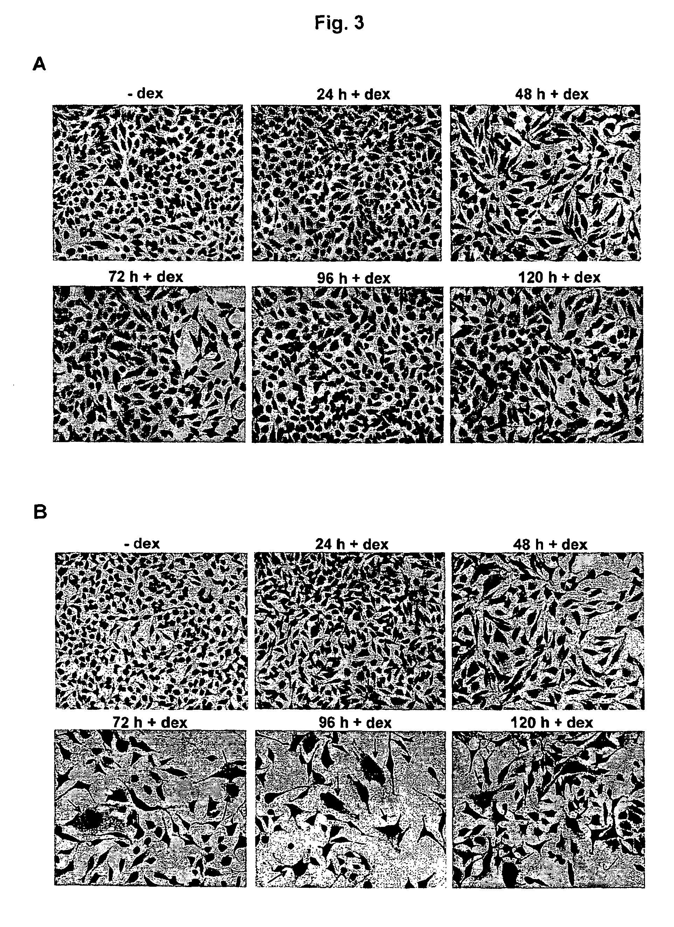

**Fig. 3** shows the morphological changes of HeLa cells stably transfected with pMMTV-holin after dexamethasone-induced λ-*S105* expression. **(A)** HeLa cells stably transfected with pMMTVb.LTR and incubated with dexamethasone for the indicated time. **(B)** HeLa cells stably transfected with pMMTV-holin and incubated with dexamethasone (+ dex) for the indicated time; - d ex, no dexamethasone treatment. Arrows point to nuclei of multinuclear cells.

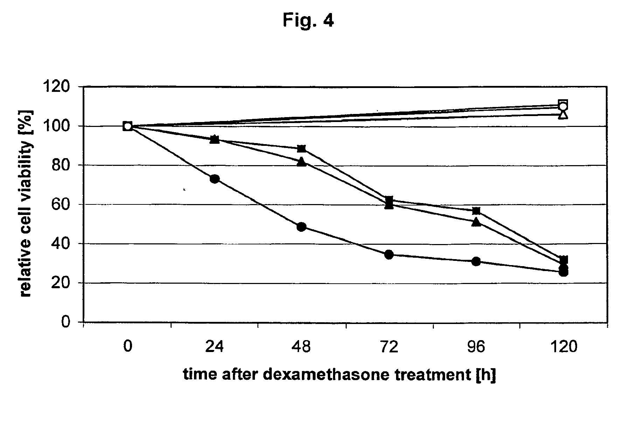

**Fig. 4** shows the effect of dexamethasone-induced λ-*S105* expression on the viability of HeLa cells as determined by measurement of the metabolic activity using a MTT test in (●) HeLa cells stably transfected with pMMTV-holin and (○) HeLa cells stably transfected with pMMTVb.LTR as a control, or by a trypan blue exclusion assay in (σ) HeLa cells stably transfected with pMMTV-holin and in (Δ) HeLa cells stably transfected with pMMTVb.LTR as a control, or by an annexinV/7AAD staining of (ν) HeLa cells stably transfected with pMMTV-holin and of 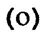 HeLa cells stably transfected with pMMTVb.LTR as a control.

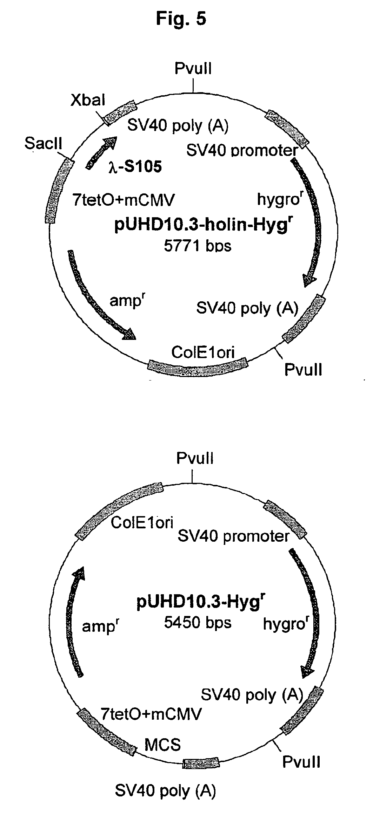

**Fig.5** shows a schematic drawing of the Tet-regulated expression vectors pUHD10.3-holin-Hyg^{r} and pUHD10.3-Hyg^{r}. SV40 poly (A), poly adenylation signal of Simian virus 40; SV40 promoter, Simian virus 40 promoter; ColE1 ori, origin of replication; amp^{r}, ampicillin resistance gene; hygro^{r}, hygromycin resistance gene; MCS, multiple cloning site; 7tetO+mCMV, 7 repeats of the tetracycline operator element coupled to a minimal promoter of human Cytomegalo virus; λ-*S105*, bacteriophage λ-holin protein encoding gene; PvuII, SacII, XbaI, restriction endonuclease binding sites.

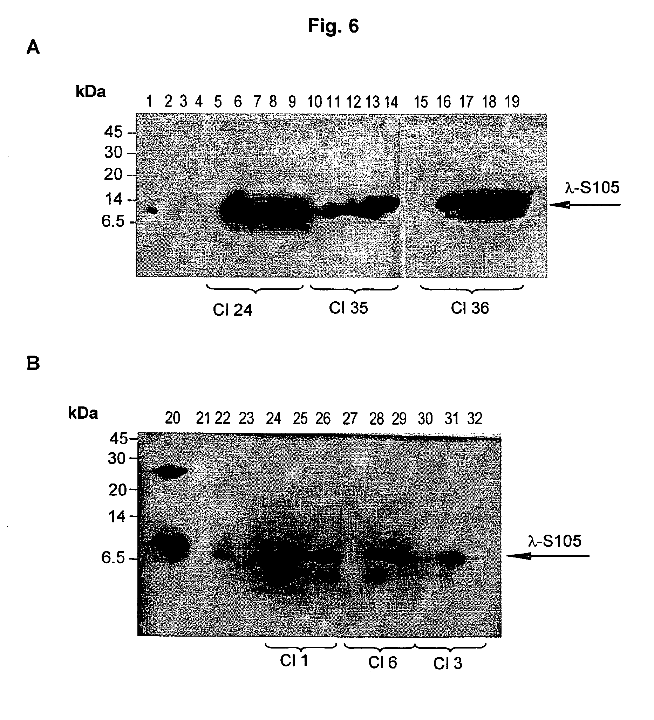

**Fig. 6** shows the expression of λ-*S105* in **(A)** HeLa cell clone CI24 (lanes 5-9), clone CI35 (lanes 10-14), clone CI36 (lanes 15-19) and **(B)** 293 cell clone CI1 (lanes 24-26), clone CI6 (lanes 27-29), clone CI3 (lane 30-32) stably transfected with pTet-On (BD Biosciences Clontech) and the expression vector pUHD10.3-holin-Hyg^{r} after stimulation with doxycycline as determined by Western blotting. The stained λ-*S105* encoded holin protein is indicated by an arrow. The sizes of the protein molecular weight standard (kDa) are given on the left. Numbering of lanes are (1, 20) lysate of *E*. *coli* inducibly expressing λ-*S105* protein; (2, 22) extracts of HeLa cells stably transfected with pMMTV-holin and treated with dexamethasone as a control; (3, 21) protein molecular weight standard; (4, 23) extracts of non-transfected HeLa cells; (5, 10, 15, 25, 27, 30) pTet-On/pUHD10.3-holin-Hyg^{r}-transfected cells not incubated with doxycycline; (6, 11, 16, 26, 28, 31); double transfected cells incubated with doxycycline for 24 h, (7, 12, 17, 24, 29, 32) for 48 h, (8, 13, 18) for 72 h, and (9, 14, 19) for 96 h.

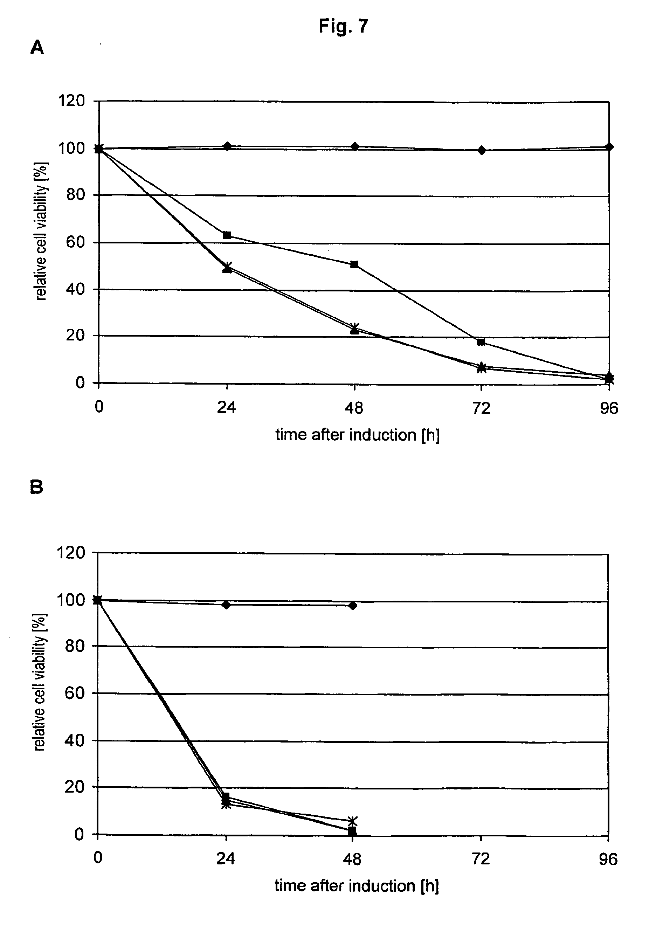

Fig. 7 shows the effect of doxycycline-induced λ-*S105* expression on cell viability in **(A)** HeLa and **(B)** 293 cell clones stably transfected with pTet-On and the expression vector pUHD10.3-holin-Hyg^{r} as determined by measurement of metabolic activity using a MTT assay. (A) ◆ non-transfected HeLa cells; ■ HeLa cell clone 24; s HeLa cell clone 35; 6 HeLa cell clone 36. (B) ◆ non-transfected 293 cells; ■ 293 cell clone 3; s 293 cell clone 6; 6 293 cell clone 1.

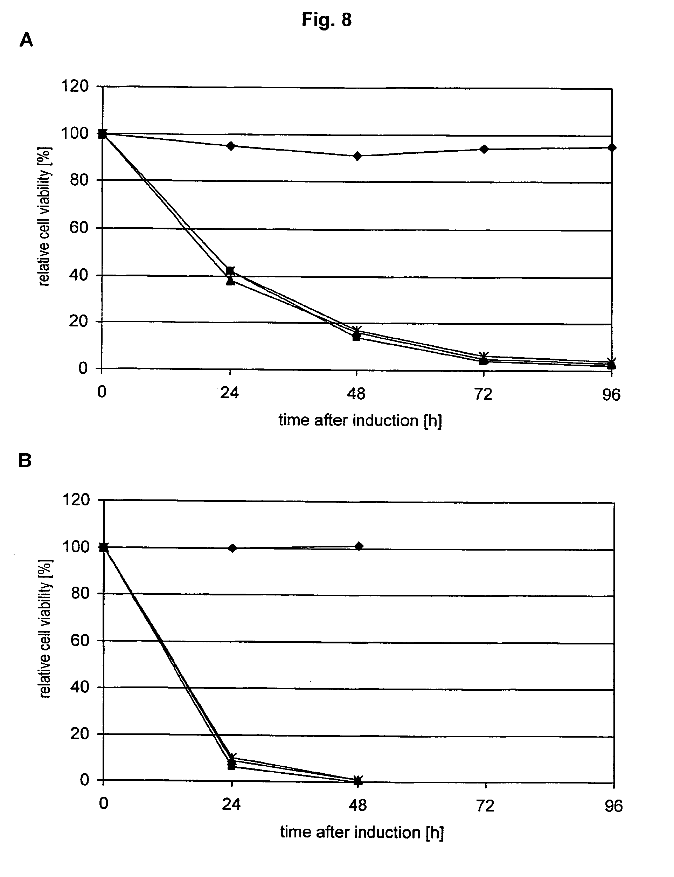

**Fig. 8** shows the effect of doxycycline-induced λ-*S105* expression on cell viability of **(A)** HeLa and **(B)** 293 cell clones stably transfected with pTet-On and pUHD10.3-holin-Hyg^{r} as determined by a trypan blue exclusion assay. **(A)** ◆ non -transfected HeLa cells; ■ HeLa cell clone 24; s HeLa cell clone 35; 6, HeLa cell clone 36. **(B)** ◆ non-transfected 293 cells; ■ 293 cell clone 3; s 293 cell clone 6; 6 293 cell clone 1.

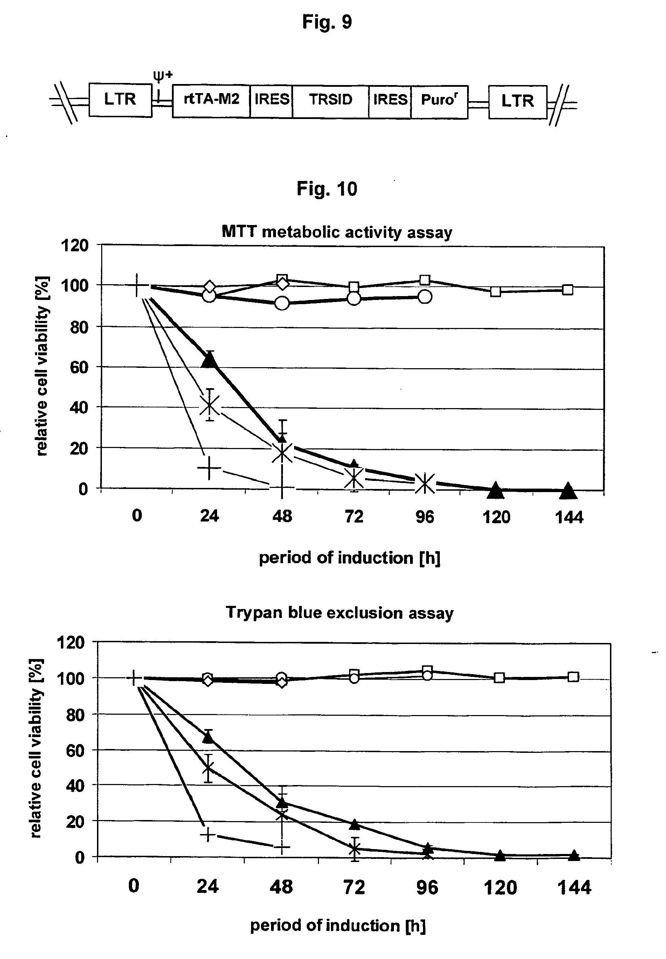

**Fig. 9** shows a retroviral vector encoding the components of a combined Tet on/Tet off regulatory system [68}. The vector pLib-rtTA-M2-IRES-TRSID-IRES-Puro^{r} is based on murine leukaemia virus (MLV). LTR, MLV long terminal repeat; IRES, internal ribosome entry site; Puro^{r}, puromycin resistance gene; Ψ+, MLV packaging signal; rtTA-M2, high affinity variant of reverse Tet-regulated transactivator (rtTA); TRSID, histone-deacetylase-reauiting Tet-repressor protein.
**Fig. 10** shows the effect of doxycycline induced expression of λ-*S105* in HeLa, 293 and MCF-7 cells stably infected with pLib-rtTA-M2-IRES-TRSID-IRES-Puro^{r} and stably transfected with pUHD10.3-holin-Hyg^{r} as determined by measurement of cell viability using MTT and trypan blue exclusion assays. ○ non-transfected MCF-7 cells; m non-transfected HeLa cells; 0 non-transfected 293 cells; s transfected/infected MCF-7 cells expressing λ-*S105*; 6 transfected/infected HeLa cells expressing λ-*S105*; + transfected/infected 293 cells expressing λ-*S105*.

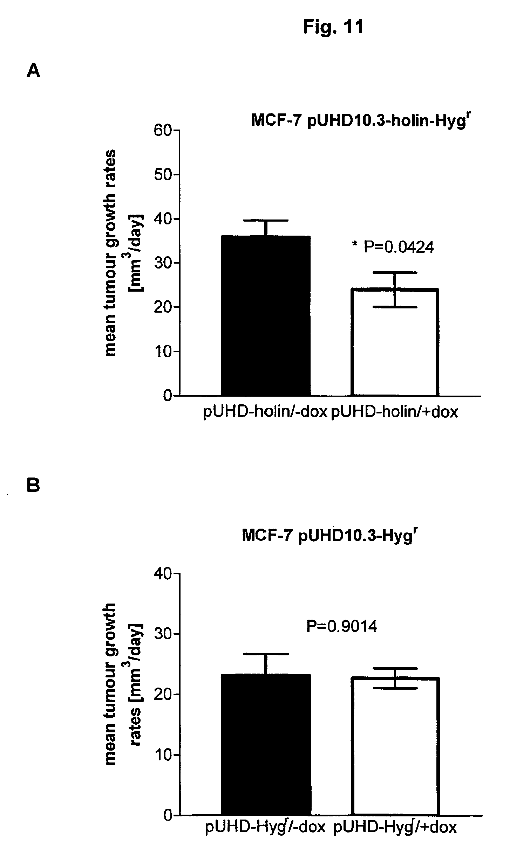

**Fig. 11** shows the effect of the λ-*S105* holin protein on human mammary tumour cell-derived xenografts in immune incompetent SCID/beige mice. MCF-7 cells harbouring the tetracyclin regulatory system and are transfected with pUHD10.3-holin-Hyg^{r} or pUHD10.3-Hyg^{r}, were injected subcutaneously into SCID/beige mice. When tumours had grown to a palpable size, mice were either given doxycycline at a concentration of 2 mg/ml (treated group) or plain drinking water (non-treated group). Tumour growth rates of doxycycline-treated and non-treated animals were compared. The differences were considered statistically significant when *p* < 0.05. **(A)** Analysis of tumour growth rates obtained with pUHD10.3-holin-Hyg^{r} transfected MCF-7 cells. Statistical evaluation indicated a significant difference (p=0.0424) of tumour growth rates between treated (+ dox) and non-treated (- dox) mice. **(B)** Comparison of growth rates of tumours obtained with pUHD10.3-Hyg^{r}-transfected MCF-7 cells. No significant difference (p=0.9014) was observed between treated (○ +dox) and non-treated (n - dox) animals.

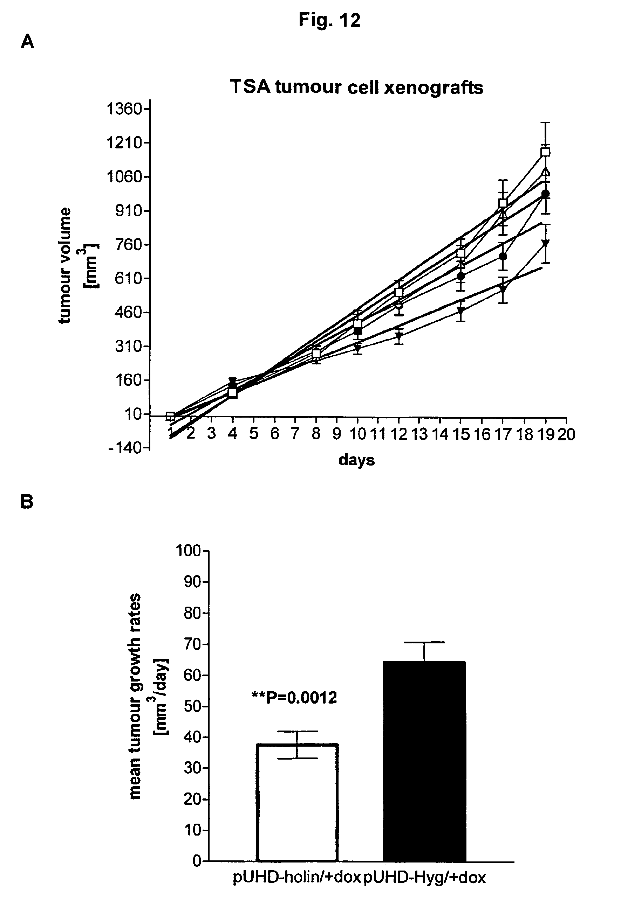

**Fig. 12** shows the effect of λ-*S105* expression on growth of murine mammary cancer cell derived xenograft tumours in a syngeneic immune competent mouse model. Murine mammary adenocarcinoma cells (TS/A) were infected with pLib-rtTA-M2-IRES-TRSID-IRES-Puro^{r} followed by transfection with pUHD10.3-holin-Hyg^{r}. A mixture of selected clones was used for establishment of subcutaneous xenograft tumours in syngeneic Balb/c mice. Infected cells transfected with pUHD10.3-Hyg^{r}were also injected. Groups were devided into 20 mice each and then treated (50 mg/kg, intraperitoneal) or not treated with doxycycline. Tumour growth was monitored and growth rates were evaluated statistically. **(A)** Tumour growth of xenografts derived from TSA cells transfected with pUHD10.3.-Hyg^{r} in non doxycycline-treated (o) and in doxycycline-treated mice (Δ) and from TSA cells transfected with pUHD10.3-holin-Hyg^{r} in non doxycycline-treated (●) and in doxycycline-treated mice (τ). **(B)** Comparison and statistical evaluation (unpaired student's *t*-test) of mean tumour growth rates of xenografts derived from pUHD10.3-holin-Hyg^{r} -transfected (○) or pUHD10.3.-Hyg^{r} transfected (v) TSA cells in doxycycline-treated mice. The difference was found to be highly significant (p=0.0012).

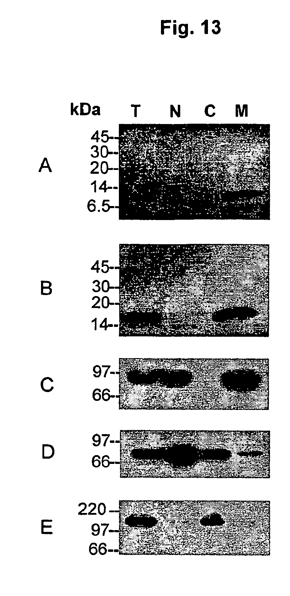

**Fig. 13** shows a Western blot analysis of subcellular fractions of cell extracts prepared from HeLa cells infected with pLib-rtTA-M2-IRES-TRSID-IRES-Puro^{r} and transfected with pUHD10.3-holin-Hyg^{r}. Cells were incubated with doxycycline for 48 h to induce λ-*S105* gene expression. Total cell lysates (T) were prepared and subjected to differential centrifugation to fractionate cytoplasm (C), nuclei (N) and mitochondria (M). Fractionated proteins were analysed by Western blotting for the presence of organelle-specific marker proteins such as cytochrome c oxidase **(B),** calnexin **(C),** nucleoporin **(D)** and integrin **(E).** Presence of the λ-*S105* protein **(A)** was detected in mitochondria and in the endoplasmic reticulum as indicated by co -localisation with cytochrome c oxidase and calnexin. The sizes of a protein molecular weight standard are depicted on the left (kDa).

The following examples serve to illustrate certain aspects of the present invention.

### EXAMPLES

### EXAMPLE 1

### Functionality of a vector expressing the phage Lambda (λ) S105 protein

In this example, the functionality of the vector used to express λ-*S105* in HeLa cells is examined. The part of the Sλ open reading frame (ORF) encoding the λ-*S105* allele (codons 3 to 107; nucleotides 45192 to 45509 of the bacteriophage λ genome [GenBank accession number NC001416]) was amplified by PCR from plasmid pVIII-S105 [53] using primers V13 and W13 (Table 1), restricted with *Xba*I and *Sac*II and ligated with the vector pMMTVGFP (Table 1) that had also been restricted with *Xba*I and *Sac*II, resulting in the vector pMMTV-holin (Fig. 1).

pMMTVGFP, the parental vector of pMMTV-holin was constructed as follows: the CMV promoter-containing *Nru*I*-Bam*HI fragment of pcDNA3 (Table 1) was replaced by *a Nru*I*-Bam*HI fragment of the vector pLTR.stp (Table 1) containing a mouse mammary tumour virus long terminal repeat (MMTV LTR), resulting in the vector pMMTVb.LTR (Table 1). The eGFP gene was cut out of pEGFP-1 (Table 1) as an *Eco*RI-*Not*I fragment and ligated with pMMTVb.LTR that had been restricted with *Eco*RI and *Not*I, giving rise to pMMTVGFP.

*E*.*coli* DH10B cells (Invitrogen) were transformed with pMMTV-holin and ampicillin resistant clones were isolated. Correct construction of the vector pMMTV-holin was confirmed by restriction enzyme analysis of prepared plasmid DNA and the integrity of the λ-*S105* gene was proven by DNA sequencing using the primer Seq-RC-MMTV-1-R (Table 1).

HeLa cells were stably transfected with pMMTV-holin using the calcium phosphate co-precipitation method according to the instructions of the manufacturer (Invitrogen) and individual clones resistant to Geneticin G418 (Invitrogen) were analysed for λ-S105 expression by Western blotting using an antiserum raised against a peptide located in the C-terminal part of the λ-*S105* encoded holin protein. pMMTV-holin allows glucocorticoid-inducible expression of λ-*S105* from the MMTV promoter located in the U3 region of the MMTV LTR. For screening of pMMTV-holin transfected cells, expression was induced by addition of 1 µM dexamethasone (Sigma-Aldrich, Vienna, Austria) to the culture medium 48 h prior to harvesting of the cells. A cell clone (clone 12) revealing inducible synthesis of the λ-*S105* protein after dexamethasone treatment was further investigated.

Therefore, 1x10⁵ cells of clone 12 or, as a control, HeLa cells stably transfected with pMMTVb.LTR, were seeded into 6-well-plates and cultivated for 120 h. At different time points, 1 µM dexamethasone was added to the culture medium and the cells were further incubated for 24, 48, 72, 96, and 120 h in the presence of the hormone. For cells, that were treated with dexamethasone for more than 48 h, fresh dexamethasone was added to the culture medium every second day. At time point 120 h, all cells were harvested simultaneously, resuspended in SDS-Laemmli sample buffer (Sigma-Aldrich, Deisenhofen, Germany) and equal amounts of protein were analysed by Western blotting using standard techniques. λ-*S105* encoded holin protein synthesis could first be detected after 24 hours of incubation with dexamethasone (Fig. 2). Expression levels thereafter stayed more or less constant for 96 h. As a control, a lysate of *E. coli* cells inducibly expressing λ-*S105* (Fig. 2, bact. lysate) was used.

The integrity of the λ-*S105* gene integrated into chromosomal DNA of stably transfected HeLa cells was confirmed by amplifying the coding sequence by PCR using the oligonucleotide primers Holin-F2 and Holin-R1 (Table 1), both binding upstream and downstream, respectively, of the λ-*S105* ORF and subsequent sequencing of the fragment with the primers Holin-F2 and Holin-R2 (Table 1).

### EXAMPLE 2

### Morphological changes of HeLa cells after dexamethasone-induced λ-S105 expression

In this example, the morphological changes of HeLa cells after dexamethasone induced λ-*S105* expression are examined. Briefly, 1x10⁴ HeLa cells stably transfected with pMMTV-holin or, as a control, pMMTVb.LTR were seeded into the chambers of 8-well chamber slides and cultivated for 120 h. At different defined time points, 1 µM dexamethasone was added to the culture medium and the cells were further incubated for 24, 48, 72, 96, and 120 h in the presence of the hormone. For cells that were treated with dexamethasone for more than 48 h, fresh dexamethasone was added to the culture medium every second day. Cells were fixed in buffered formalin and counterstained for 2 min in hemalum solution (Fluka, Seelze, Germany). Slides were analysed by standard light microscopy (Zeiss Axiovert 200M, Carl Zeiss GmbH, Vienna, Austria) and photographed. While pMMTVb.LTR-transfected cells did not show any significant change in their morphology after dexamethasone treatment (Fig. 3A), dexamethasone-induced expression of λ-*S105* in pMMTV-holin-transfected HeLa cells with time provoked a kind of "swelling", eventually resulting in huge, often multinuclear cells (Fig. 3B, arrows). In addition, in comparison to the respective controls stably transfected with pMMTVb.LTR (Fig. 3A) cell numbers continuously decreased in pMMTV-holin-transfected cells starting 48 h after induction of λ-*S105* expression (Fig. 3B, 48 h + dex, 72 h + dex, 96 h + dex, 120 h + dex).

### EXAMPLE 3

### Effect of dexamethasone-induced λ-S105 expression on cellular metabolism

In this example, the effect of dexamethasone-induced λ-*S105* expression in HeLa cells on cellular metabolism as determined by the measurement of relative NADH and NADPH levels using a MTT assay (Sigma-Aldrich, Vienna, Austria) is described. Briefly, 1x10⁵ HeLa cells stably transfected with pMMTV-holin or, as a negative control, pMMTVb.LTR were seeded into 6-well-plates and cultivated for 120 h. At different defined time points, 1 µM dexamethasone was added to the culture medium and the cells were incubated for 24, 48, 72, 96 and 120 h in the presence of the hormone. For cells that were treated with dexamethasone for more than 48 h, fresh dexamethasone was added to the culture medium every second day. At time point 120 h, all cells were harvested simultaneously and subjected to a MTT assay for determining the relative amounts of NADH and NADPH as an indicator for cell metabolism which, in turn, is an indicator for cell viability. For HeLa cells stably transfected with pMMTV-holin, incubation with dexamethasone for 120 h resulted in a significant decrease of viable cells by 74% when compared to cells that were not treated with dexamethasone (Fig. 4, filled circlets), In order to exclude a negative effect of dexamethasone itself on cell growth and viability, HeLa cells stably transfected with pMMTVb.LTR lacking the λ-*S105* encoding region were also incubated with dexamethasone for 120 h (Fig. 4, empty circles). When viable cell numbers of HeLa cells stably transfected with pMMTV-holin and grown for 120 h but not incubated with dexamethasone (filled circle at time point 0 h in Fig. 4) are set to 100% and compared to HeLa cells stably transfected with pMMTVb.LTR but otherwise treated in the same way (empty circle at time point 120 h in Fig. 4), it is obvious, that dexamethasone had no negative effect on cell growth. In contrast, HeLa cells lacking the λ-*S105* gene revealed a higher number of viable cells (110%) when compared to cells harboring the gene (100%). This effect can be explained by the leakiness of the MMTV promoter system and a constitutive low level expression of λ*-S105* in cells transfected with pMMTV-holin even in the absence of dexamethasone or due to low levels of glucocorticoids present in the cell culture medium. Taken together, these results demonstrate that λ-*S105* expression has a pronounced negative effect on cell viability as indicated by a decrease of cellular metabolism.

### EXAMPLE 4

### Effect of dexamethasone-induced λ-S105 expression on membrane integrity

In this example, the effect of dexamethasone-induced λ-*S105* expression in HeLa cells on membrane integrity as determined by applying a trypan blue exclusion assay is described. Leakiness of the cytoplasmic membrane is a well-known indicator for cell death by apoptosis or necrosis. 1x10⁵ HeLa cells stably transfected with pMMTV-holin or, as a control, pMMTVb.LTR were seeded into 6-well-plates and cultivated for 120 h. At different defined time points, 1 µM dexamethasone was added to the culture medium and the cells were further incubated for 24, 48, 72, 96, and 120 h in the presence of the hormone. For cells, that were treated with dexamethasone for more than 48 h, fresh dexamethasone was added to the culture medium every second day. At time point 120 h, all cells were harvested simultaneously and subjected to trypan blue staining to determine the amount of viable cells with intact membranes. The trypan blue exclusion assay was performed according to the instructions of the manufacturer (Sigma-Aldrich, Vienna, Austria). For HeLa cells stably transfected with pMMTV-holin, incubation with dexamethasone for 120 h resulted in a significant lower number of cells with an intact (trypan blue-excluding) membrane (30%) when compared to cells that were not treated with dexamethasone and that were set to 100% (Fig. 4, filled triangles). In order to exclude a negative effect of dexamethasone itself on membrane integrity, HeLa cells stably transfected with pMMTVb.LTR lacking λ-*S105* were also incubated with dexamethasone for 120 h. When the numbers of non-compromised HeLa cells stably transfected with pMMTV-holin that had been grown for 120 h but had not been incubated with dexamethasone (100%; filled triangle at time point 0 h in Fig. 4) are compared to HeLa cells stably transfected with pMMTVb.LTR but otherwise treated in the same way (empty triangle at time point 120 h in Fig. 4), it is obvious, that dexamethasone itself did not cause the membrane damage. In contrast, HeLa cells lacking the λ-*S105* gene revealed a higher number of viable cells with an intact cytoplasmic membrane (106%) when compared to cells harbouring the gene (100%). As also mentioned in example 3, this effect is most likely due to the leakiness of the MMTV promoter system. Taken together, the results described in this example again demonstrate the harmful effect of the λ-*S105* encoded holin protein on cellular viability as shown by a loss of cytoplasmic membrane integrity.

### EXAMPLE 5

### Effect of dexamethasone-induced λ-S105 expression on the cytoplasmic membrane

In this example, the effect of dexamethasone-induced expression of the λ-*S105* protein on the cytoplasmic membrane as determined by annexin V-PE/7AAD staining is described. Annexin V is the natural binding partner for phosphatidylserine (PS) located in the inner membrane leaflets which face the cytosol. However, during apoptosis of cells, PS is exposed at the cell surface and can therefore be detected by annexin V added from outside. Binding of annexin V to cells is an indicator for apoptosis but also necrosis since cells with compromised membranes will allow annexin V to enter and bind PS from inside (55). 7AAD (7-amino-actinomycin D) intercalates into doublestranded nucleic acids. It is excluded by viable cells with intact membranes but can penetrate cell membranes of late apoptotic and/or necrotic cells.

1x10⁵ HeLa cells stably transfected with pMMN-holin or pMMTVb.LTR lacking the λ-*S105* encoding region were seeded into 6-well-plates and cultivated for 120 h. At different defined time points, 1 µM dexamethasone was added to the culture medium and the cells were further incubated for 24, 48, 72, 96, and 120 h in the presence of the hormone. For cells, that were treated with dexamethasone for more than 48 h, fresh dexamethasone was added to the culture medium every second day. At time point 120 h, all cells were harvested simultaneously and subjected to annexinV-PE/7AAD staining according to the instructions of the manufacturer (BD Biosciences, Heidelberg, Germany). When the number of cells that had not been treated with dexamethasone and did not show annexin V-PE/7AAD staining (therefore regarded as living cells) are set to 100% and compared to the number of annexin V-PE/7AAD-negative cells of the pool of cells that had been treated with dexamethasone for 120 h, it becomes obvious that much less cells (32%) are viable (Fig. 4). In order to investigate a possible effect of dexamethasone itself on membrane integrity, HeLa cells stably transfected with pMMTVb.LTR lacking the λ-*S105* gene were also incubated with dexamethasone for 120 h. When the numbers of HeLa cells stably transfected with pMMTV-holin that had been grown for 120 h but had not been incubated with dexamethasone (100%; filled squares at time point 0 h in Fig. 4) are compared to HeLa cells stably transfected with pMMTVb.LTR but otherwise treated in the same way (empty squares at time point 120 h in Fig. 4), it is obvious, that dexamethasone itself did not cause the membrane damage. As also pointed out in examples 3 and 4, HeLa cells lacking the λ-*S105* gene revealed a higher number of viable cells after 120 h of dexamethasone treatment (111%) compared to cells harboring the gene (100%). Again, this effect is most likely due to the leakiness of the MMTV promoter system (see example 3). Taken together, this example demonstrates the powerful cell killing effect of λ-*S105* when expressed in HeLa cells.

### EXAMPLE 6

### Evaluation of a doxycycline-induced vector system

In this example, the effect of λ-*S105* expression in HeLa and 293 cells using a doxycycline-inducible Tet-On gene expression system (BD Biosciences, Heidelberg, Germany) is described. For this purpose cells were stably transfected with pTet-On followed by transfection with the respective λ-*S105* encoding Tet-response vector pUHD10.3-holin Hyg^{r} or the parental vector pUHD10.3-Hyg^{r} (Fig. 5). The tetracycline response plasmid pUHD-10.3-holin was constructed by inserting an amplified DNA fragment of 318 bp in length containing the λ-*S105* encoding region (NCBI-Gene bank accession number J02459; nucleotides 45192-45509) into plasmid pUHD-10.3 [http://www2.cbm.uam.es/bc-015/seauenoes/pUHD10-3.html]. The primers used for amplification (V13 and W13 [Table 1]) comprise *Sac*II and *Xba*I sites for enzymatic digestion and ligation of the obtained fragment with the *Xba*I and *Sac*II sites of a 3.15 kb vector fragment of pUHD-10.3. The hygromycin resistance gene expression cassette (2.3 kb) was obtained from pcDNA3-Hyg^{r} (Invitrogen LT, Lofer, Austria) by restriction enzyme digestion with Pvull. The 2.3 kb fragment was ligated with the Pvull-linearised vector pUHD-10.3-holin and pUHD-10.3, respectively, giving rise to the vectors pUHD-10.3-holin-Hyg^{r} and pUHD10.3-Hyg^{r} (Fig. 5).

Stably transfected cell clones were analysed for doxycycline-inducible λ-*S105* expression by Western blotting using an antiserum raised against a peptide located in the C-terminal part of the λ-*S105* encoded holin protein. Among the λ-*S105* expressing clones, the three clones showing highest expression levels were further analysed. HeLa cells were cultivated in the presence of 5 µg/ml doxycycline for 24, 48, 72, and 96 h. 293 cells were cultivated only for 24 and 48 h since all cells were dead after this incubation period. λ-*S105* expression in HeLa cell clones 24, 35, 36 and in 293 cell clones 1, 3, and 6 is shown in Fig. 6A and B, respectively. While no (Fig. 6, lanes 5, 15, 27, 30) or low (Fig. 6, lanes 10, 25) background expression was observed in HeLa and 293 control cells that had not been incubated with doxycycline, very high expression was observed already 24 h after addition of doxycycline to the culture medium (Fig. 6 lanes 6, 11, 16, 26, 28, 31). As a control, the same cell lysate of E. *coli* cells expressing λ-*S105* described in example 1 (Fig. 6 lanes 1 and 20) as well as a cell extract of HeLa cells stably expressing λ-*S105* from vector pMMTV-holin (Fig. 6 lanes 2 and 22; see example 1) was used. Compared to dexamethasone-induced HeLa cells expressing λ-*S105* from vector pMMTV-holin (Fig. 6B, lane 22), the expression level was dramatically enhanced by expressing λ-*S105* with the Tet-On vector system. The low amount of λ-*S105* holin protein detected in extracts of 293 cell clone 3 after 48 h of incubation with doxycycline by Western blotting (Fig. 6B, lane 32) is due to the fact that most of the cells had already died at this time point. The larger protein band stained in lane 20 (Fig. 6B) containing the bacterial lysate most probably represents an oligomeric form of λ-*S105* while the smaller proteins (Fig. 6B, lanes 24, 25, 26, 28) detected beyond the stained λ-holin protein (marked by an arrow) may indicate partial degradation of the λ*-S105 holin* protein in 293 cells.

### EXAMPLE 7

### Effect of doxycycline-induced λ-S105 expression on cellular metabolism

This example describes the effect of doxycycline-induced Tet-regulated λ-*S105* expression in HeLa and 293 cells on the cellular metabolism as determined by the measurement of relative NADH and NADPH levels as an indicator for cell viability using a MTT assay. Stably transfected HeLa cell clones 24, 35, 36 and 293 cell clones 1, 3, and 6 harbouring the λ-*S105* -containing Tet-regulated expression vector pUHD10.3-holin-Hyg^{r} were cultivated in the presence of 5 µg/ml doxycycline for 24, 48, 72, and 96 h (HeLa cell clones) or 24 and 48 h (293 cell clones) to induce λ-*S105* expression. λ-*S105* encoded holin synthesis was verified by Western blotting (see example 6). Cells were subjected to a MTT assay (see example 3) for determining cell viabilities (Fig. 7). Viability of non-transfected HeLa (Fig. 7A) and 293 (Fig. 7B) cells lacking the vector (Fig. 7, black diamonds) were not impaired demonstrating that doxycycline itself had no negative effect on the cells. However, expression of λ-*S105* in pUHD10.3-holin-Hyg^{r}-transfected 293 and HeLa and cell clones (Fig. 7, filled squares, triangles, crosses) resulted in a substantial cell killing of 94% to 98% after 48 h to 96 h of incubation with doxycycline.

### EXAMPLE 8

### Effect of doxycycline-induced λ-S105 expression on cytoplasmic membrane integrity

In this example, the effect of doxycycline-induced λ-*S105* expression in HeLa and 293 cells on the cytoplasmic membrane integrity as an indicator of cell viability as determined by trypan blue staining is described. λ-*S105* protein was expressed using a doxycycline-inducible Tet-On gene expression system (see example 7). HeLa cell clones 24, 35, 36 and 293 cell clones 1, 3, and 6 harbouring the Tet-regulated expression vector pUHD10.3-holin-Hyg^{r} were examined. λ-*S105* expression was induced by adding 5 µM doxycycline and cells were cultivated in the presence of doxycycline for 24, 48, 72, and 96 h (HeLa cell clones) or 24 and 48 h (293 cell clones). λ-*S105* protein expression was verified by Western blotting (see example 6). Trypan blue staining (see example 4) was performed according to the instructions of the manufacturer (Sigma-Aldrich, Vienna, Austria). For HeLa (Fig. 8A) and 293 cells (Fig. 8B) stably transfected with pUHD10.3-holin-Hyg^{r}, incubation with doxycycline for 96 h resulted in a decrease of viable cells of 97% to 100% (Fig. 8, filled squares, triangles, crosses) whereas HeLa and 293 cells lacking the vector (Fig. 8, filled diamonds) showed no loss of viability after doxycycline treatment. This obvious damage of the cytoplasmic membrane indicated by a failure in trypan blue exclusion may either be due to apoptotic or necrotic cell death mediated by λ-*S105* expression.

### EXAMPLE 9

### Evaluation of a Tet on/off regulated expression of λ-S105 on cell viability of eukaryotic cancer cell lines

In order to obtain an absolutely tight regulated expression system for synthesis of the λ**-***S105* holin protein in eukaryotic cancer cells a Tet on/off vector system was established. For this purpose the retroviral vector pLib-rtTA-M2-IRES-TRSID-IRES-Puro^{r} (Fig. 9), which is based on a murine leukaemia virus (MLV), was constructed as described in [68]. Briefly, the reverse Tet-regulated transactivator (rtTA) high affinity variant rtTA2-M2 was cloned into a modified pLIB retroviral vector (BD Biosciences, Heidelberg) further encoding a histone-deacetylase-recruiting Tet-repressor protein (TRSID) and a puromycin resistance gene (Puro^{r}). Each coding region is separated by an internal ribosome entry site (IRES) which allows simultaneous translation of all three proteins from one mRNA which is driven from the retroviral promoter located with the 5' long terminal repeat (LTR). Vectors combining the high-affinity rtTA variant (rtTA2-M2) with a TRSID protein showed a greatly diminished leakiness which makes them especially useful for tightly controlled expression of cytotoxic genes. Human cervix (HeLa) and mammary carcinoma (MCF-7) cell lines as well as embryonic kidney cells (293) were infected with recombinant vector viruses generated from 293-deprived amphotropic packaging cell lines [71]. Selected cell populations were further transfected with pUHD10.3-holin-Hyg^{r} (Fig. 5) and single clones expressing λ-*S105* upon stimulation with doxycycline were further investigated.

Therefore, 3x10⁵ MCF-7 or 1x10⁵ HeLa or 2x10⁵ 293 cells/well were seeded into 6-well plates. The next day, 5 µg/ml doxycycline was added to the cell culture medium. For cells that were treated with doxycycline for more than 24 h, fresh doxycycline was added to the cell culture medium daily and cells were incubated for an additional 24, 48, 72, 96, and 120 h. At the time point 144 h, all cells were simultaneously harvested. The floating and the adherent cells from each sample were pooled and harvested together. The total cell number (cells/well) was determined by a CasyTT® cell counter (Schärfe System GmbH, Reutlingen, Germany). For evaluation of the effects of induced λ-holin protein synthesis on cell viability, MTT (Fig. 10, upper panel) and trypan blue exclusion assays (Fig. 10, lower panel) were performed (see examples 7 and 8). Cell killing ratios (% viability) were defined as the ratio between the doxycycline-induced and non-induced cells. Both assays revealed a severe decrease of cell viability upon stimulation of λ*-S105* gene expression by doxycycline in all three cell lines (Fig. 10). No cytotoxic effect was noticed, when non-transfected cells were treated with doxycycline (Fig. 10, open symbols). Dependent on the cell line, a 90% cell death was observed between 48 and 96 h of doxycycline-induced λ*-S105* expression.

### EXAMPLE 10

### Evaluation of λ-S105 gene expression on the growth of human mammary tumour cell xenografts in immune incompetent SCID/beige mice

For evaluation of the cytotoxic activity of the λ-*S105* encoded holin protein *in vivo,* 5x10⁶ MCF -7 cells that stably harboured the Tet-regulated λ-holin encoding plasmid pUHD10.3-holin-Hyg^{r} or the respective basal vector pUHD10.3-Hyg^{r} were injected subcutaneously into the mammary fat pad of female SCID/beige mice (C.B-17/lcrHsd-Prkcd*^{scid}* Lyst*^{bg}*; Harlan-Winkelmann, Bochen, Germany). Tumour sizes were measured twice a week in two dimensions with a caliper and the tumour volume was calculated according to the formula [I x w x w/2]. 37 days post implantation, when the tumours had grown to approximately 50 mm³, a 1.7 mg 17β-estradiol (E2) 90-day slow-release pellet (Innovative Research, FL, USA) was implanted into each mouse to further enhance tumour growth. After tumours had grown to a palpable size (∼200 mm³), animals were divided into two groups. One group was orally administered with doxycycline dissolved in drinking water containing 5% sucrose to a final concentration of 2 mg/ml, while the other group obtained sucrose-enriched drinking water lacking doxycycline. Animals were treated with doxycycline for 32 days. At the end of the experiment or when the tumour size exceeded 1800 mm³, mice were sacrificed and dissected. Tumour growth rates were calculated as the mean slopes of tumour growth curves of each individual animal (Fig. 11). A significantly reduced tumour growth was observed when doxycycline-treated mice bearing pUHD10.3-holin-Hyg^{r}-transfected MCF-7 cells were compared to animals that did not obtain doxycycline (Fig. 11A, p=0.0424). In contrast, no difference in tumour growth was obtained with animals bearing MCF-7 cells transfected with the parental vector pUHD10.3-Hyg^{r} in response to doxycycline treatment (Fig. 11B).

### EXAMPLE 11

### Effect of doxycycline-induced λ-S105 gene expression on tumour growth in a syngeneic mammary adenocarcinoma mouse model

For evaluation of the anti-tumorigenic potential of the λ-*S105* encoded holin protein in an immune competent model, murine mammary adenocarcinoma cells (TSA) were established containing the Tet on/off regulated pUHD10.3-holin-Hyg^{r} expression system or the parental vector pUHD10.3-Hyg^{r}. 1x10⁶ cells were injected subcutaneously into the mammary fat pad of syngeneic Balb/c mice. 7 days later mice were inoculated daily with doxycycline (50 mg/kg body weight, i.p.). Control groups consisting of randomly chosen mice did not obtain doxycycline. Tumour growth of all animals was monitored twice a week and tumour volumes were calculated according to the formula [I x w x w/2] (Fig. 12A). As shown in Fig. 12B, a highly significant reduced tumour growth (p=0.0012) was observed when doxycycline-treated animals that harboured λ*-S105* expressing TSA cells (white bar) were compared to animals containing pUHD10.3-Hyg^{r}-transfected cells (black bar).

### EXAMPLE 12

### Determination of the intracellular localisation of the λ-S105 encoded holin protein in eukaryotic cells by sub-cellular fractionation

For investigation of subcellular localisation of the λ-holin protein 1x10⁷ HeLa cells that stably harboured the Tet on/off regulated expression vector pUHD10.3-holin-Hyg^{r} were incubated for 24 h with 5 µg/ml doxycycline. After treatment, cells were gently scraped off from the culture flask and harvested by centrifugation at 250xg for 5 min. Subcellular fractionation was performed as described [70] with minor modifications. Briefly, cells were resuspended in 5 vol. of ice-cold buffer A containing 20 mM Hepes-KOH, pH 7.5, 10 mM KCI, 1.5 mM MaCl₂, 1 mM EDTA, 1 mM EGTA, 1 mM dithiothreitol (DTT), protease inhibitor cocktail (Sigma-Aldrich, Deisenhofen, Germany) and 250 mM sucrose. After allowing cells to swell for 10 min, they were lysed by 15-20 passages through a 25-gauge needle, and homogenates were washed five times with ice cold buffer A by centrifugation at 1000xg for 10 min at 4°C to isolate the nuclei (fraction N). The supernatant was centrifuged at 10000xg for 15 min at 4°C. The resulting mitochondria-enriched pellets (fraction M) were washed five times with ice cold buffer A. The resulting supernatant was used as the cytosolic fraction (C). 15-30 µg of protein - total cellular extract (T) and sub-cellular fractions - in Laemmli-sample buffer containing 10% β-mercaptoethanol (Sigma-Aldrich, Deisenhofen, Germany) were separated by SDS polyacrylamide gel electrophoresis and analysed by Western blotting (Fig. 13). Membranes were probed either with polyclonal rabbit anti-holin antiserum or antibodies detecting organelle-specific marker proteins such as cytochrome c oxidase (Cox IV) (1:50) (Eubio, Vienna, Austria), calnexin (1:50) (Becton Dickinson, Vienna, Austria), nucleoporine (1:50) (Becton Dickinson, Vienna, Austria) and integrin α2 (1:25) (Becton Dickinson, Vienna, Austria). Finally, proteins were detected by enhanced chemiluminescence (Amersham Biosciences). The λ-holin protein (Fig. 13A) was shown to co-localize in sub-cellular fractions containing Cox IV (Fig. 13B) and calnexin (Fig. 13C) indicating its presence in mitochondria and the endoplasmic reticulum, respectively. Holin protein was not detected in fractions containing nuclei or cytoplasmic membranes as determined by the presence of nucleoporine (Fig. 13D) and integrine α2 (Fig. 13E), respectively.

### REFERENCES CITED

1. Aghi M, Hochberg F, Breakefield XO: Prodrug activation enzymes in cancer gene therapy. J Gene Med, 2000. 2(3),148-64.
2. Moolten FL: Tumor chemosensitivity conferred by inserted herpes thymidine kinase genes: paradigm for a prospective cancer control strategy. Cancer Res, 1986. 46(10) 5276-81.
3. Danielsen S, Kilstrup M, Barilla K, Jochimsen B, Neuhard J: Characterization of the Escherichia coli codBA operon encoding cytosine permease and cytosine deaminase. Mol Microbiol, 1992. 6(10), 1335-44.
4. Weber G F, Waxman DJ: Activation of the anti-cancer drug ifosphamide by rat liver microsomal P450 enzymes. Biochem Pharmacol, 1993. 45(8), 1685-94.
5. Wei MX, Tamiya T, Chase M, Boviatsis EJ, Chang TK, Kowall NW, Hochberg FH, Waxman DJ, Breakefield XO, Chiocca EA: Experimental tumor therapy in mice using the cyclophosphamide-activating cytochrome P450 2B1 gene. Hum Gene Ther, 1994. 5(8), 969-78.
6. Rigg A, Sikora K: Genetic prodrug activation therapy. Mol Med Today, 1997. 3(8),. 359-66.
7. Igney FH, Krammer PH: Death and anti-death: tumour resistance to apoptosis. Nat Rev Cancer, 2002. 2(4), 277-88.
8. Schwartz PS, Waxman DJ: Cyclophosphamide induces caspase 9-dependent apoptosis in 9L tumor cells. Mol Pharmacol, 2001. 60(6), 1268-79.
9. Diaz RM, Bateman A, Emiliusen L, Fielding A, Trono D, Russell SJ, Vile RG: A lentiviral vector expressing a fusogenic glycoprotein for cancer gene therapy. Gene Ther, 2000. 7(19), 1656-63.
10. Fielding AK, Chapel-Fernandes S, Chadwick MP, Bullough FJ, Cosset FL, Russell SJ: A hyperfusogenic gibbon ape leukemia envelope glycoprotein: targeting of a cytotoxic gene by ligand display. Hum Gene Ther, 2000. 11(6), 817-26.
11. Galanis E, Bateman A, Johnson K, Diaz RM, James CD, Vile R, Russell SJ: Use of viral fusogenic membrane glycoproteins as novel therapeutic transgenes in gliomas. Hum Gene Ther, 2001. 12(7), 811-21.
12. Bateman A, Bullough F, Murphy S, Emiliusen L, Lavillette D, Cosset FL, Cattaneo R, Russell SJ, Vile RG: Fusogenic membrane glycoproteins as a novel class of genes for the local and immune-mediated control of tumor growth. Cancer Res, 2000. 60(6), 1492-7.
13. Bateman AR, Harrington KJ, Kottke T, Ahmed A, Melcher AA, Gough MJ, Linardakis E, Riddle D, Dietz A, Lohse CM, Strome S, Peterson T, Simari R, Vile RG: Viral fusogenic membrane glycoproteins kill solid tumor cells by nonapoptotic mechanisms that promote cross presentation of tumor antigens by dendritic cells. Cancer Res, 2002. 62(22), 6566-78.
14. Higuchi H, Bronk SF, Bateman A, Harrington K, Vile RG, Gores GJ: Viral fusogenic membrane glycoprotein expression causes syncytia formation with bioenergetic cell death: implications for gene therapy. Cancer Res, 2000. 60(22), 6396-402.
15. Lidor YJ, Lee WE, Nilson JH, Maxwell IH, Su LJ, Brand E, Glode LM: In vitro expression of the diphtheria toxin A-chain gene under the control of human chorionic gonadotropin gene promoters as a means of directing toxicity to ovarian cancer cell lines. Am J Obstet Gynecol, 1997. 177(3), 579-85.
16. Massuda ES, Dunphy EJ, Redman RA, Schreiber JJ, Nauta LE, Barr FG, Maxwell IH, Cripe TP: Regulated expression of the diphtheria toxin A chain by a tumor-specific chimeric transcription factor results in selective toxicity for alveolar rhabdomyosarcoma cells. Proc Natl Acad Sci USA, 1997. 94(26), 14701-6.
17. Maxwell IH, Maxwell F,Glode FM: Expression of diphtheria toxin A-chain in mature B-cells: a potential approach to therapy of B-lymphoid malignancy. Leuk Lymphoma, 1992. 7(5-6), 457-62.
18. Maxwell IH, Maxwell F,Glode FM: Regulated expression of a diphtheria toxin A-chain gene transfected into human cells: possible strategy for inducing cancer cell suicide. Cancer Res, 1986. 46(9), 4660-4.
19. Allam M, Bertrand R, Zhang-Sun G, Pappas J, Viallet J: Cholera toxin triggers apoptosis in human lung cancer cell lines. Cancer Res, 1997. 57(13), 2615-8.
20. Kiura K, Ohnoshi T, Ueoka H, Takigawa N, Tabata M, Segawa Y, Shibayama T, Kimura I: Inhibitory effects of cholera toxin on in vitro growth of human lung cancer cell lines. Anticancer Drug Des, 1993. 8(6), 417-28.
21. Bodart JF, Chopra A, Liang X, Duesbery N: Anthrax, MEK and cancer. Cell Cycle, 2002. 1(1), 10-5.
22. Frankel AE, Powell BL, Duesbery NS, Vande Woude GF, Leppla SH: Anthrax fusion protein therapy of cancer. Curr Protein Pept Sci, 2002. 3(4), 399-407.
23. Liu S, Bugge TH, Leppla SH: Targeting of tumor cells by cell surface urokinase plasminogen activator-dependent anthrax toxin. J Biol Chem, 2001. 276(21), 17976-84.
24. Liu S, Aaronson H, Mitola DJ, Leppla SH, Bugge TH: Potent antitumor activity of a urokinase-activated engineered anthrax toxin. Proc Natl Acad Sci U S A, 2003. 100(2), 657-62.
25. Weber E., Anderson WF, Kasahara N: Recent advances in retrovirus vector-mediated gene therapy: teaching an old vector new tricks. Curr Opin Mol Ther, 2001. 3(5), 439-53.
26. Cosset FL, Takeuchi Y, Battini JL, Weiss RA, Collins MK.: High-titer packaging cells producing recombinant retroviruses resistant to human serum. J Virol, 1995. 69(12), 7430-6.
27. Saier, M.H., Jr.: Families of proteins forming transmembrane channels. J Membr Biol, 2000. 175(3), 165-80.
28. Young R, Bläsi U: Holins: form and function in bacteriophage lysis. FEMS Microbiol Rev, 1995. 17(1-2), 191-205.
29. Wang IN, Smith DL, Young R: Holins: the protein clocks of bacteriophage infections. Annu Rev Microbiol, 2000. 54, 799-825.
30. Barenboim, M., et al., Characterization of the dual start motif of a class II holin gene. Mol Microbiol, 1999. 32(4), 715-27.
31. Bläsi U., et al., The C-terminal sequence of the lambda holin constitutes a cytoplasmic regulatory domain. J Bacterial, 1999. 181(9), 2922-9.
32. Zagotta MT, Wilson DB: Oligomerization of the bacteriophage lambda S protein in the inner membrane of Escherichia coli. J Bacterial, 1990. 172(2), 912-21.
33. Smith DL, Struck DK, Scholtz JM, Young R: Purification and biochemical characterization of the lambda holin. J Bacteriol, 1998. 180(9), 2531-40.
34. Chang CY, Nam K, Young R: S gene expression and the timing of lysis by bacteriophage lambda. J Bacteriol, 1995. 177(11), 3283-94.
35. Bläsi U , Young R: Two beginnings for a single purpose: the dual-start holins in the regulation of phage lysis. Mol Microbiol, 1996. 21(4), 675-82.
36. Steiner, M, Lubitz W , Bläsi U: The missing link in phage lysis of gram-positive bacteria: gene 14 of Bacillus subtilis phage phi 29 encodes the functional homolog of lambda S protein. J Bacteriol, 1993. 175(4), 1038-42.
37. Tedin K, Resch A, Steiner M, Blasi U: Dual translational start motif evolutionarily conserved in the holin gene of Bacillus subtilis phage phi 29. Virology, 1995. 206(1), 479-84.
38. Gründling A, Smith DL, Bläsi U, Young R: Dimerization between the holin and holin inhibitor of phage lambda. J Bacterial, 2000. 182(21), 6075-81.
39. Wang IN, Deaton J, Young R: Sizing the holin lesion with an endolysin-beta-galactosidase fusion. J Bacterial, 2003. 185(3), 779-87.
40. Young, KD, Young R: Lytic action of cloned phi X174 gene E. J Virol, 1982. 44(3), 993-1002.
41. Henrich B, Lubitz W, Plapp R: Lysis of Escherichia coli by induction of cloned phi X174 genes. Mol Gen Genet, 1982. 185(3), 493-7.
42. Winter RB, Gold L: Overproduction of bacteriophage Q beta maturation (A2) protein leads to cell lysis. Cell, 1983. 33(3), 877-85.
43. Witte A, Brand E, Mayrhofer P, Narendja F, Lubitz W: Mutations in cell division proteins FtsZ and FtsA inhibit phiX174 protein-E-mediated lysis of Escherichia coli. Arch Microbiol, 1998. 170(4), 259-68.
44. Witte A, Bläsi U, Halfmann G, Szostak M, Wanner G, Lubitz W: Phi X174 protein E-mediated lysis of Escherichia coli. Biochimie, 1990. 72(2-3), 191-200.
45. Roof WD, Home SM, Young KD, Young R: slyD, a host gene required for phi X174 lysis, is related to the FK506- binding protein family of peptidyl-prolyl cis-trans-isomerases. J Biol Chem, 1994. 269(4), 2902-10.
46. Bernhardt T G, Roof WD, Young R: The Escherichia coli FKBP-type PPlase SlyD is required for the stabilization of the E lysis protein of bacteriophage phi X174. Mol Microbiol, 2002. 45(1), 99-108.
47. Bernhardt TG, Roof WD, Young R: Genetic evidence that the bacteriophage phi X174 lysis protein inhibits cell wall synthesis. Proc Natl Acad Sci U S A, 2000. 97(8), 4297-302.
48. Bernhardt, TG, Struck DK, Young R: The lysis protein E of phi X174 is a specific inhibitor of the MraY catalyzed step in peptidoglycan synthesis. J Biol Chem, 2001. 276(9), 6093-7.
49. Bernhardt TG, Wang IN, Struck DK, Young R: A protein antibiotic in the phage Qbeta virion: diversity in lysis targets. Science, 2001. 292(5525), 2326-9.
50. Witte A, Wanner G, Bläsi U, Halfmann G, Szostak M, Lubitz W: Endogenous transmembrane tunnel formation mediated by phi X174 lysis protein E. J Bacterial, 1990. 172(7),4109-14.
51. Garrett J, Bruno C, Young R: Lysis protein S of phage lambda functions in Saccharomyces cerevisiae. J Bacteriol, 1990. 172(12), 7275-7.
52. Tabotta, W, Klein D, Hohenadl C, Salmons B, Günzburg WH. Genetic reshuffling reconstitutes functional expression cassettes in retroviral vectors. J Gene Med, 2001. 3(5), 418-26.
53. Graschopf A, Bläsi U: Molecular function of the dual-start motif in the lambda S holin. Mol Microbiol, 1999. 33(3), 569-82.
54. Bläsi U, Nam K, Hartz D, Gold L,Young R: Dual translational initiation sites control function of the lambda S gene. EMBO J, 1989. 8(11), 3501-10.
55. Koopman G, Reutelingsperger CP, Kuijten GA, Keehnen RM, Pals ST, van Oers MH: Annexin V for flow cytometric detection of phosphatidylserine expression on B cells undergoing apoptosis. Blood. 1994. 84(5),1415-20
56. Plumb JA, Bilsland A, Kakani R, Zhao J, Glasspool RM, Knox RJ, Evans TRJ, Keith WN. Telomerase-specific suicide gene therapy vectors expressing bacterial nitroreductase sensitize human cancer cells to the pro-drug CB1954. Oncogene, 2001, 20, 7797-803
57. Cortes ML, de Felipe P, Martin V, Hughes MA, lzquierdo M: Successful use of a plant gene in the treatment of cancer in vivo. Gene Ther, 1998, 5, 1499-1507
58. Martin V, Cortes ML, de Felipe P, Farsetti A, Calcaterra NB, lzquierdo M: Cancer gene therapy by thyroid hormone-mediated expression of toxin genes Cancer Res, 2000, 60(12), 3218-3224
59. Kondo S, Bama BP, Morimura T, Takeuchi J, Yuan J, Akbasak A, Bamett GH: Interleukin 1β-converting enzyme mediates cisplatin-induced apoptosis in malignant glioma cells. Cancer Res, 1995, 55(24), 173-180
60. Kondo S, Tanaka Y, Kondo Y, lshizaka Y, Hitomi M, Haqqi T, Liu J, Barnett GH, Alnemri ES, Bama BP: Retroviral transfer of CPP32β gene into malignant gliomas in vitro and in vivo. Cancer Res. 1998, 58(5), 962-967
61. Kondo S, Ishizaka Y, Okada T, Kondo Y, Hitomi M, Tanaka Y, Haqqi T, Barnett GH, Barna BP: FADD gene therapy for malignant gliomas in vitro and in vivo. Hum. Gene Ther. 1998, 9(11): 1599-1608
62. Shinoura N, Koike H, Furitu T, Hashimoto M, Asai A, Kirino T, Hamada H: Adenovirus-mediated transfer of caspase-8 augments cell death in gliomas: implication for gene therapy. Hum. Gene Ther. 2000, 11(8), 1123-37
63. Shinoura N, Saito K, Yoshida Y, Hashimoto M, Asai A, Kirino T, Hamada H: Adenovirus-mediated transfer of bax with caspase-8 controlled by myelin basic protein promoter exerts an enhanced cytotoxic effect in gliomas. Cancer Gene Ther. 2000, 7(5), 739-48
64. Jaggi R, Salmons B, Müllener D, Groner B: The v-mos and H-ras oncogene expression represses glucocorticoid hormone-dependent transcription from the mouse mammary tumor virus LTR. Embo J. 1986, 5(10), 2609-16
65. Thackray VG, Lieberman BA, Nordeen SK: Differential gene induction by glucocorticoid and progesterone receptors. J. Steroid Biochem. Mol. Biol. 1998, 66,171-78
66. Berens C, Hillen W: Gene regulation by tetracyclines: Constraints of resistance regulation in bacteria shape TetR for application in eukaryotes. Eur J Biochem 2003, 270, 3109-21
67. Jiang W, Zhou L, Breyer B, Feng T, Cheng H, Haydon R, Ishikawa A, He TC: Tetracycline-regulated Gene Expression Mediated by a Novel Chimeric Repressor That Recruits Histone Deacetylases in Mammalian Cells. J Biol Chem 2001, 276(48), 45168-74
68. Ausserlechner MJ, Obexer, P, Kofler R: Dose-dependent Control of Retroviral Gene Expression by Tricistronic Tetracyclin-regulated Transactivator/Repressor Vectors*.*
69. Gossen M. and Bujard H.: Tight control of gene expression in mammalian cells by tetracycline-responsive promoters. Proc. Natl Acad. Sci. USA, 1992, 89, 5547-51
70. Cuvillier O, Edsall L, Spiegel S: Involvement of sphingosine in mitochondria-dependent Fas-induced apoptosis of type II Jurkat T cells. J Biol Chem. 2000, 275(21):15691-700.
71. Pambalk K, Hohenadl C, Salmons B, Gunzburg WH, Renner M: Specific packaging of spliced retroviral vector transcripts lacking the Psi-region. Biochem Biophys Res Commun. 2002, 293(1), 239-46.
72. Nanni P, de Giovanni C, Lollini PL, Nicoletti G, Prodi G: TS/A: a new metastasizing cell line from a BALB/c spontaneous mammary adenocarcinoma. Clin Exp Metastasis. 1983, 1(4), 373-80.

**Table 1: Bacterial Strains, Human Cell Lines, Plasmids and Primers**

| **Bacterial Strains /** | **Human Cell Lines** | **Source/Reference** |
|---|---|---|
| *E.coli* DH10B | | Invitrogen |
| HeLa | | ATCC (No. CCL-2) |
| HEK 293 | | ATCC (No. CRL-1573) |
| MCF-7 | | ATCC (No. HTB-22) |
| TS/A | | www.biotech.ist.unige.it/ |
| | | cldb/cl4545.html [72] |

| **Plasmids** | | **Source/Reference** |
|---|---|---|
| pTet-On | | BD Biosciences-Clontech |
| pLTR-stp | | [64] |
| pVIII-S105 | | [53] |
| pLS105 | | [54] |
| pLib-rtTA-M2-IRES-TRSID-IRES-Puro^{r} | | [68] |
| pcDNA3-hyg^{r} | | Invitrogen |
| pEGFP-1 | | BD Biosciences |
| pUHD-10.3 | | http://www2.cbm.uam.es/bc-015/ |
| | | sequences/pUHD10-3.html |
| pUHD-10.3-holin | | present invention |
| pUHD-10.3-hyg^{r} | | present invention |
| pUHD-10.3-holin-hyg^{r} | | present invention |
| pMMTV-holin | | present invention |
| pMMTVb.LTR | | present invention |
| pMMTVGFP | | present invention |

| **Primers** | **Nucleotide Sequence** | **SEQ ID Number** |
|---|---|---|
| V13 | 5' AAAAAACCGCGGATGCCAGAAAAACATGACCTGTTGGCC 3' (SEQ ID No. 1) | |
| W13 | 5' AAAAAATCTAGATTATTATTGATTTCTACCATCTTCTACTCC 3' (SEQ ID No. 2) | |
| Holin-F2 | 5' CGCCAGTGTGCTGGAATTCT 3' (SEQ ID No. 3) | |
| Holin-R1 | 5' CTGGCAACTAGAAGGCACAG 3' (SEQ ID No. 4) | |
| Holin-R2 | 5' AAGGCACAGTCGAGGCTGAT 3' (SEQ ID No. 5) | |
| Seq-RC-MMTV-1-R | 5' ACGAGGATGTGAGACAAGTG 3' (SEQ ID No. 6) | |

### SEQUENCE LISTING

<110> Fonds zur Foerderung der Forschung auf dem Gebiet der molekularen Virologie und Gentherapie
<120> Bacteriophage and Prophage Proteins in Cancer Gene Therapy
<130> P-FON-06-PCT
<160> 9
<170> PatentIn version 3.1
<210> SEQ ID No 1
   <211> 39
   <212> DNA
   <213> Artificial Sequence
   <220>
   <223> Primer
<400> 1
   aaaaaaccgc ggatgccaga aaaacatgac ctgttggcc 39
<210> SEQ ID No 2
   <211> 42
   <212> DNA
   <213> Artificial Sequence
   <220>
   <223> Primer
<400> 2
   aaaaaatcta gattattatt gatttctacc atcttctact cc 42
<210> SEQ ID No 3
   <211> 20
   <212> DNA
   <213> Artificial Sequence
<220>
   <223> Primer
<400> 3
   cgccagtgtg ctggaattct 20
<210> SEQ ID No 4
   <211> 20
   <212> DNA
   <213> Artificial Sequence
<220>
   <223> Primer
<400> 4
   ctggcaacta gaaggcacag 20
<210> SEQ ID No 5
   <211> 20
   <212> DNA
   <213> Artificial Sequence
<220>
   <223> Primer
<400> 5
   aaggcacagt cgaggctgat 20
<210> SEQ ID No 6
   <211> 20
   <212> DNA
   <213> Artificial Sequence
<220>
   <223> Primer
<400> 6
   acgaggatgt gagacaagtg 20
<210> SEQ ID No 7
   <211> 105
   <212> PRT
   <213> Bacteriophage lambda
   <308> P03705
   <313> (3) .. (107) 
Met Pro Glu Lys Asn Asp Leu Leu Ala Ala Ile Leu Ala Ala Lys Glu
1               5                   10                  15

Gln Gly Ile Gly Ala Ile Leu Ala Phe Ala Met Ala Tyr Leu Arg Gly
            20                  25                  30

Arg Tyr Asn Gly Gly Ala Phe Thr Lys Thr Val Ile Asp Ala Thr Met
        35                  40                  45

Cys Ala Ile Ile Ala Trp Phe Ile Arg Asp Leu Leu Asp Phe Ala Gly
    50                  55                  60

Leu Ser Ser Asn Leu Ala Tyr Ile Thr Ser Val Phe Ile Gly Tyr Ile
65                  70                  75                  80

Gly Thr Asp Ser Ile Gly Ser Leu Ile Lys Arg Phe Ala Ala Lys Lys
                85                  90                  95

Ala Gly Val Glu Asp Gly Arg Asn Gln
            100                 105
<210> SEQ ID No 8
   <211> 107
   <212> PRT
   <213> Bacteriophage lambda
   <308> P03705
   <313> (1) .. (107) 
Met Lys Met Pro Glu Lys Asn Asp Leu Leu Ala Ala Ile Leu Ala Ala
1               5                   10                  15

Lys Glu Gln Gly Ile Gly Ala Ile Leu Ala Phe Ala Met Ala Tyr Leu
            20                  25                  30

Arg Gly Arg Tyr Asn Gly Gly Ala Phe Thr Lys Thr Val Ile Asp Ala
        35                  40                  45

Thr Met Cys Ala Ile Ile Ala Trp Phe Ile Arg Asp Leu Leu Asp Phe
    50                  55                  60

Ala Gly Leu Ser Ser Asn Leu Ala Tyr Ile Thr Ser Val Phe Ile Gly
65                  70                  75                  80

Tyr Ile Gly Thr Asp Ser Ile Gly Ser Leu Ile Lys Arg Phe Ala Ala
                85                  90                  95

Lys Lys Ala Gly Val Glu Asp Gly Arg Asn Gln
            100                 105
<210> SEQ ID No 9
   <211> 131
   <212> PRT
   <213> Bacteriophage phi-29
   <308> P11188
   <313> (1)..(131)

Met Lys Met Ile Ala Trp Met Gln His Phe Leu Glu Thr Asp Glu Thr
1               5                   10                  15

Lys Leu Ile Tyr Trp Leu Thr Phe Leu Met Val Cys Met Val Val Asp
            20                  25                  30

Thr Val Leu Gly Val Leu Phe Ala Lys Leu Asn Pro Asn Ile Lys Phe
        35                  40                  45

Ser Ser Phe Lys Ile Lys Thr Gly Val Leu Ile Lys Val Ser Glu Met
    50                  55                  60

Ile Leu Ala Leu Leu Ala Ile Pro Phe Ala Val Pro Phe Pro Ala Gly
65                  70                  75                  80

Leu Pro Leu Leu Tyr Thr Val Tyr Thr Ala Leu Cys Val Ser Glu Ile
                85                  90                  95

Tyr Ser Ile Phe Gly His Leu Arg Leu Val Asp Asp Lys Ser Asp Phe
            100                 105                 110

Leu Glu Ile Leu Glu Asn Phe Phe Lys Arg Thr Ser Gly Lys Asn Lys
        115                 120                 125

Glu Glu Lys
                    130

## Claims

1. An isolated bacteriophage-derived protein for the use in the treatment of a proliferative disease or disorder, wherein the bacteriophage-derived protein induces a cell growth inhibitory activity or cell killing effect in eukaryotic cells.

2. The isolated bacteriophage-derived protein according to claim 1, wherein the bacteriophage-derived protein is an alpha-helix-type channel forming protein or at least the functional part or analoga thereof.

3. The isolated bacteriophage-derived protein according to claim 1 or 2,, wherein the bacteriophage-derived protein is a holin.

4. The isolated bacteriophage-derived protein according to any of the claims 1 to 3, wherein the bacteriophage-derived protein is selected from the group consisting of Lambda S105 and Lambda S107 protein (Swiss-Prot Accession Number P03705), the Phi29 GP 14 protein (Swiss-Prot Accession Number P11188), the phage T4 Immunity protein (Swiss-Prot Accession Number P08986), the phage P22 protein 13 (Swiss-Prot Accession Number P09962), the phage P2 TM protein Y (Swiss-Prot Accession Number P51773), the M protein of phage PRD1 (Swiss-Prot Accession Number P27389), the holin protein of phage A118 (Swiss-Prot Accession Number Q37975), the phage A500 holin protein (Swiss-Prot Accession Number Q37977), the DPH holin protein of phage Dp-1 (Swiss-Prot Accession Number 003978), the holin protein of phage HP1 (Swiss-Prot Accession Number P51727), the holin protein of phage rlt (Swiss-Prot Accession Number Q38134), the lysis protein 17.5 of phages T7 (Swiss-Prot Accession Number P03802) and T3 (Swiss-Prot Accession Number P10307), the NucE holin protein of phage P2 Ogr (Swiss-Prot Accession Number Q54418), the holin protein isolated from *Haemophilus somnus* (Swiss-ProtAccession Number Q48281), the P10 protein of phage Phi-6 (Swiss-Prot Accession Number P11127), the protein rV of phage T4 (Swiss-Prot Accession Number P06808), the *Staphylococcus* phage Phi-11 holin (Swiss-Prot Accession Number Q38020), and the *Lactococcus* phage Tuc2009 holin (Swiss-Prot Accession Number Q38613).

5. The isolated bacteriophage-derived protein for the use according to claims 1 to 4, wherein the eukaryotic cell is a mammalian and/or human cell.

6. The isolated bacteriophage-derived protein according to any of the claims 1 to 5" wherein the bacteriophage-derived protein is transduced into the eukaryotic cell by a eukaryotic expression vector.

7. The isolated bacteriophage-derived protein for the use according to claim 6, wherein the eukaryotic expression vector is a viral vector, particularly a retroviral vector, more particularly a replication competent retroviral vector, a ProCon or a ReCon vector.

8. Viral expression vector useful according to claims 6 or 7 comprising and expressing from an operably linked regulatory element the nucleic acid sequence encoding a bacteriophage-derived protein, which induces a cell growth inhibitory activity or cell killing effect in eukaryotic cells.

9. Viral expression vector comprising and expressing from an operably linked regulatory element a fusion protein with a bacteriophage-derived protein, which induces a cell growth inhibitory activity or cell killing effect in eukaryotic cells.

10. Viral expression vector according to claim 8 or 9, wherein the vector is a retroviral vector, more particularly a replication competent retroviral vector, a ProCon or a ReCon vector.

11. Nucleic acid sequence comprising the sequence encoding the viral expression vector according to any of the claims 8 to 10.

12. A viral particle containing the viral expression vector according to any of the claims 8 to 10.

13. Eukaryotic cell comprising the nucleic acid sequence according to claim 11.

14. Eukaryotic cell according to claim 13 capable of producing viral particles according to claim 12.

15. An in vitro method for introducing the nucleic acid sequence according to claim 11 into an eukaryotic cell, said method comprising the step transducing an eukaryotic cell with the expression vector according to any of the claims 8 to 10; or infecting an eukaryotic cell with the viral particle according to the claim 12.

16. A composition comprising a therapeutically effective amount of the viral vector according to any of the claims 8 to 10, the nucleic acid sequence according to claim 11, the viral particle according to claim 12, the eukaryotic cell according to claim 14, an isolated bacteriophage-derived protein and/or a fusion protein with a bacteriophage-derived protein, which induces a cell growth inhibitory activity or cell killing effect, and a pharmaceutically acceptable carrier.

17. A composition comprising the viral vector according to any of the claims 8 to 10, the nucleic acid sequence according to claim 11, the viral particle according to claim 12, the eukaryotic cell according to claim 14, an isolated bacteriophage-derived protein and/or a fusion protein with a bacteriophage-derived protein encapsulated in a porous membrane, which allows after administration to a patient a liberation of a therapeutically effective amount of said viral vector, said nucleic acid sequence, said viral particle, said eukaryotic cell, said bacteriophage-derived protein and/or said fusion protein with a bacteriophage-derived protein from the capsules, and a pharmaceutically acceptable carrier.

18. Use of the viral vector according to any of the claims 8 to 10, the nucleic acid sequence according to claim 11, the viral particle according to claim 12, the eukaryotic cell according to claim 14, an isolated bacteriophage-derived protein, a fusion protein with a bacteriophage-derived protein, which induces a cell growth inhibitory activity or cell killing effect and/or a composition according to claim 16 or 17 as medicament or for the manufacture of a medicament for treatment of cancer diseases.

## Patentansprüche

1. Isoliertes, von Bakteriophagen stammendes Protein zur Verwendung in der Behandlung einer proliferativen Erkrankung oder Störung, wobei das von Bakteriophagen stammende Protein eine zellwachstumshemmende Aktivität oder eine zelltötende Wirkung in eukaryontischen Zellen auslöst.

2. Isoliertes, von Bakteriophagen stammendes Protein gemäß Anspruch 1, wobei das von Bakteriophagen stammende Protein ein kanalbildendes Protein vom Alpha-Helix-Typ oder wenigstens der funktionelle Teil oder Analoga desselben sind.

3. Isoliertes, von Bakteriophagen stammendes Protein gemäß Anspruch 1 oder 2, wobei das von Bakteriophagen stammende Protein ein Holin ist.

4. Isoliertes, von Bakteriophagen stammendes Protein gemäß einem der Ansprüche 1 bis 3, wobei das von Bakteriophagen stammende Protein aus der Gruppe ausgewählt ist, die aus Lambda-S105- und Lambda-S107-Protein (Swiss-Prot-Akzessionsnummer P03705), dem Phi29-GP-14-Protein (Swiss-Prot-Akzessionsnummer P11188), dem Phagen-T4-Immunitätsprotein (Swiss-Prot-Akzessionsnummer P089986), dem Phagen-P22-Protein 13 (Swiss-Prot-Akzessionsntirmner P09962), dem Phagen-P2-TM-Protein Y (Swiss-Prot-Akzessionsnummer P51773), dem M-Protein des Phagen PRD1 (Swiss-Prot-Akzessionsnummer P27389), dem Holinprotein des Phagen A118 (Swiss-Prot-Akzessionsnummer Q37975), dem Phagen-A500-Holinprotein (Swiss-Prot-Akzessionsnummer Q37977), dem DPH-Holinprotein des Phagen Dp-1 (Swiss-Prot-Akzessionsnunmer 003978), dem Holinprotein des Phagen HP1 (Swiss-Prot-Akzessionsnummer P51727), dem Holinprotein des Phagen rlt (Swiss-Prot-Akzessionsnummer Q38134), dem Lysisprotein 17.5 der Phagen T7 (Swiss-Prot-Akzessionsnummer P03802) und T3 (Swiss-Prot-Akzessionsnummer P10307), dem NucE-Holinprotein des Phagen P2 Ogr (Swiss-Prot-Akzessionsnummer Q54418), dem aus *Haemophilus somnus* isolierten Holinprotein (Swiss-Prot-Akzessionsnummer Q48281), dem P10-Protein des Phagen Phi-6 (Swiss-Prot-Akzessionsnummer P11127), dem Protein rV des Phagen4 (Swiss-Prot-Akzessionsnummer P06808), dem *Staphylococcus*-Phagen Phi-11-Holin (Swiss-Prot-Ahessionsnummer Q38020) und dem *Lactococcus*-Phagen Tuc2009-Holin (Swiss-Prot-Akzessionsnummer Q38613) besteht.

5. Isoliertes, von Bakteriophagen stammendes Protein zur Verwendung gemäß Anspruch 1 bis 4, wobei die eukaryontische Zelle eine Säugetier- und/oder Humanzelle ist.

6. Isoliertes, von Bakteriophagen stammendes Protein gemäß einem der Ansprüche 1 bis 5, wobei das von Bakteriophagen stammende Protein durch einen eukaryontischen Expressionsvektor in die eukaryontische Zelle transduziert ist.

7. Isoliertes, von Bakteriophagen stammendes Protein zur Verwendung gemäß Anspruch 6, wobei der eukaryontische Expressionsvektor ein viraler Vektor, insbesondere ein retroviraler Vektor, insbesondere ein replikationskompetenter retroviraler Vektor, ein ProCon- oder ein ReCon-Vektor ist.

8. Gemäß Anspruch 6 oder 7 verwendbarer viraler Expressionsvektor, der die Nukleinsäuresequenz, welche ein von Bakteriophagen stammendes Protein kodiert, das eine zellwachstumshemmende Aktivität oder zelltötende Wirkung in eukaryontischen Zellen auslöst, umfasst und aus einem operabel verknüpften regulatorischen Element exprimiert.

9. Viraler Expressionsvektor, der ein Fusionsprotein mit einem von Bakteriophagen stammenden Protein, das eine zellwachstumshemmende Aktivität oder zelltötende Wirkung in eukaryontischen Zellen auslöst, umfasst und aus einem operabel verknüpften regulatorischen Element exprimiert.

10. Viraler Expressionsvektor gemäß Anspruch 8 oder 9, wobei der Vektor ein retroviraler Vektor, insbesondere ein replikationskompetenter retroviraler Vektor, ein ProCon-oder ein ReCon-Vektor ist.

11. Nukleinsäuresequenz, welche die Sequenz umfasst, die den viralen Expressionsvektor gemäß einem der Ansprüche 8 bis 10 kodiert.

12. Viruspartikel, das den viralen Expressionsvektor gemäß einem der Ansprüche 8 bis 10 enthält.

13. Eukaryontische Zelle, welche die Nukleinsäuresequenz gemäß Anspruch 11 umfasst.

14. Eukaryontische Zelle gemäß Anspruch 13, die in der Lage ist, Viruspartikel gemäß Anspruch 12 zu produzieren.

15. In-Vitro-Verfahren zum Einführen der Nukleinsäuresequenz gemäß Anspruch 11 in eine eukaryontische Zelle, wobei das Verfahren den Schritt umfasst, eine eukaryontische Zelle mit dem Expressionsvektor gemäß einem der Ansprüche 8 bis 10 zu transduzieren oder eine eukaryontische Zelle mit dem Viruspartikel gemäß Anspruch 12 zu infizieren.

16. Zusammensetzung, die eine therapeutisch wirksame Menge des viralen Vektors gemäß einem der Ansprüche 8 bis 10, der Nukleinsäuresequenz gemäß Anspruch 11, des Viruspartikels gemäß Anspruch 12, der eukaryontischen Zelle gemäß Anspruch 14, eines isolierten, von Bakteriophagen stammenden Proteins und/oder eines Fusionsproteins mit einem von Bakteriophagen stammenden Protein, welches eine zellwachstumshemmende Aktivität oder zelltötende Wirkung auslöst, und einen pharmazeutisch akzeptablen Träger umfasst.

17. Zusammensetzung, die den viralen Vektor gemäß einem der Ansprüche 8 bis 10, die Nukleinsäuresequenz gemäß Anspruch 11, das Viruspartikel gemäß Anspruch 12, die eukaryontische Zelle gemäß Anspruch 14, ein isoliertes, von Bakteriophagen stammendes Protein und/oder ein Fusionsprotein mit einem von Bakteriophagen stammenden Protein, eingekapselt in eine poröse Membran, die nach der Verabreichung an einen Patienten eine Freisetzung einer therapeutisch wirksamen Menge des viralen Vektors, der Nukleinsäuresequenz, des Viruspartikels, der eukaryontischen Zelle, des von Bakteriophagen stammenden Proteins und/oder des Fusionsproteins mit einem von Bakteriophagen stammenden Protein aus den Kapseln erlaubt, und einen pharmazeutisch akzeptablen Träger umfasst.

18. Verwendung des viralen Vektors gemäß einem der Ansprüche 8 bis 10, der Nukleinsäuresequenz gemäß Anspruch 11, des Viruspartikels gemäß Anspruch 12, der eukaryontischen Zelle gemäß Anspruch 14, eines isolierten, von Bakteriophagen stammenden Proteins, eines Fusionsproteins mit einem von Bakteriophagen stammenden Protein, das eine zellwachstumshemmende Aktivität oder zelltötende Wirkung auslöst, und/oder einer Zusammensetzung gemäß Anspruch 16 oder 17 als Medikament oder zur Herstellung eines Medikamentes zur Behandlung von Krebserkrankungen.

## Revendications

1. Une protéine dérivée d'un bactériophage isolée pour l'utilisation dans le traitement d'une maladie ou d'un trouble prolifératif où la protéine dérivée de bactériophages induit une activité d'inhibition de croissance de cellule ou un effet d'élimination de cellule dans les cellules eucaryotes.

2. La protéine dérivée d'un bactériophage isolée selon la revendication 1, où la protéine dérivée de bactériophages est une protéine formant un canal de type hélice alpha ou au moins la partie fonctionnelle ou l'analogue à celle-ci.

3. La protéine dérivée d'un bactériophage isolée selon la revendication 1 ou 2 où la protéine dérivée de bactériophages est une holine.

4. La protéine dérivée d'un bactériophage isolée selon l'une des revendications 1 à 3 où la protéine dérivée d'un bactériophage est sélectionnée parmi le groupe constitué de Lambda S105 et Lambda S107 protein (Swiss-Prot Accession Number P03705), Phi29 GP 14 protein (Swiss-Prot Accession Number P11188), phage T4 Immunity protein (Swiss-Prot Accession Number P08986), phage P22 protein (Swiss-Prot Accession Number P09962), phage P2 TM protein Y (Swiss-Prot Accession Number P51773), M protein of phage PRD1 (Swiss-Prot Accession Number P27389), holin protein of phage A118 (Swiss-Prot Accession Number Q37975), phage A500 holin protein (Swiss-Prot Accession Number Q37977), DPH holin protein of phage Dp-1 (Swiss-Prot Accession Number 003978), holin protein of phage HP 1 (Swiss-Prot Accession Number P51727), holin protein of phage rlt (Swiss-Prot Accession Number Q38134), lysis protein 17,5 of phages T7 (Swiss-Prot Accession Number P03802) et T3 (Swiss-Prot Accession Number P 10307), NucE holin prpotein of phage P2 Pgr (Swiss-Prot Accession Number Q54418), holin protein isolated from Haemophilus somnus (Swiss-Prot Accession Number Q48281), P10 protein of phage Phi-P6 (Swiss-Prot Accession Number P11127), protein rV of phage T4 (Swiss-Prot Accession Number P06808), Staphylococcus phage Phi-11 holin (Swiss-Prot Accession Number Q38020) et Lactococcus phage Tuc2009 holin (Swiss-Prot Accession Number Q38613).

5. La protéine dérivée d'un bactériophage isolée selon les revendications 1 à 4 où la cellule eucaryote est une cellule mammifère et/ou humaine.

6. La protéine dérivée d'un bactériophage isolée selon l'une des revendications 1 à 5, où la protéine dérivée d'un bactériophage est transduite en une cellule eucaryote par un vecteur d'expression eucaryote.

7. La protéine dérivée d'un bactériophage isolée pour l'utilisation selon la revendication 6 où le vecteur d'expression eucaryote est un vecteur viral, particulièrement un vecteur rétroviral plus particulièrement un vecteur rétroviral apte à la réplication, un vecteur ProCon ou ReCon.

8. Un vecteur viral d'expression utile selon les revendications 6 ou 7 comprenant la séquence d'acide nucléique encodant une protéine dérivée d'un bactériophage qui induit une activité d'inhibition de croissance de cellule ou un effet d'élimination de cellule dans les cellules eucaryotes, et l'exprimant depuis un élément de régulation lié de manière.

9. Un vecteur viral d'expression comprenant et exprimant depuis un élément régulateur lié de manière fonctionnelle une protéine de fusion avec une protéine dérivée d'un bactériophage qui induit une activité d'inhibition de croissance de cellule ou un effet d'élimination de cellule dans les cellules eucaryotes.

10. Un vecteur viral d'expression selon la revendication 8 ou 9 où le vecteur est un vecteur rétroviral, plus particulièrement un vecteur rétroviral apte à la réplication, un vecteur ProCon ou ReCon.

11. Une séquence d'acide nucléique comprenant la séquence encodant le vecteur viral d'expression selon l'une des revendications 8 à 10.

12. Une particule virale contenant le vecteur viral d'expression selon l'une des revendications 8 à 10.

13. Une cellule eucaryote comprenant la séquence d'acide nucléique selon la revendication 11.

14. Une cellule eucaryote selon la revendication 13 capable de produire des particules virales selon la revendication 12.

15. Un procédé in vitro pour introduire la séquence d'acide nucléique selon la revendication 11 dans une cellule eucaryote, ledit procédé comprenant l'étape de transduction d'une cellule eucaryote avec le vecteur d'expression selon l'une des revendications 8 à 10, ou d'infection d'une cellule eucaryote avec la particule virale selon la revendication 12.

16. Une composition comprenant un montant thérapeutiquement efficace de vecteur viral selon l'une des revendications 8 à 10, la séquence d'acide nucléique selon la revendication 11, la particule virale selon la revendication 12, la cellule eucaryote selon la revendication 14, une protéine dérivée d'un bactériophage isolée et/ou une protéine de fusion avec une protéine dérivée de bactériophage qui induit une activité d'inhibition de croissance de cellule ou un effet d'élimination de cellule et un porteur acceptable pharmaceutiquement.

17. Une composition comprenant le vecteur viral selon l'une des revendications 8 à 10, la séquence d'acide nucléique selon la revendication 11, la particule virale selon la revendication 12, la cellule eucaryote selon la revendication 14, une protéine dérivée d'un bactériophage isolée et/ou une protéine de fusion avec une protéine dérivée d'un bactériophage encapsulée dans une membrane poreuse qui permet après administration à un patient une libération d'un montant efficace thérapeutiquement dudit vecteur viral, ladite séquence d'acide nucléique, ladite particule virale ladite cellule eucaryote, ladite protéine dérivée d'un bactériophage et/ou la protéine de fusion avec une protéine dérivée d'un bactériophage depuis les capsules et un porteur acceptable pharmaceutiquement.

18. Utilisation du vecteur viral selon l'une des revendications 8 à 10, la séquence d'acide nucléique selon la revendication 11, la particule virale selon la revendication 12, la cellule eucaryote selon la revendication 14, une protéine dérivée d'un bactériophage isolée, une protéine de fusion avec une protéine dérivée d'un bactériophage qui induit une activité d'inhibition de croissance de cellule ou un effet d'élimination de cellule et/ou une composition selon la revendication 16 ou 17 comme médicament ou pour la fabrication d'un médicament pour le traitement de maladie cancéreuse.
